# EUROPEAN PATENT SPECIFICATION

(11) **EP 2 734 149 B1**
(45) Date of publication and mention of the grant of the patent: **07.09.2016**
(21) Application number: 12815414.3
(22) Date of filing: 19.07.2012
(51) Int. Cl.: A61F 2/04

(54) **DEVICES AND METHODS FOR TREATING SLEEP DISORDERED BREATHING**
VORRICHTUNGEN UND VERFAHREN ZUR BEHANDLUNG VON ATEMSTÖRUNGEN IM SCHLAF
DISPOSITIFS ET PROCÉDÉS DE TRAITEMENT DE RESPIRATION AFFECTÉE PAR DES TROUBLES DU SOMMEIL

(30) Priority: 21.07.2011 US 201161510315 P
(43) Date of publication of application: 28.05.2014
(73) Proprietor: Svip 7 Llc, Palo Alto, CA 94301 (US)
(72) Inventor: SHALON, Tidhar, Palo Alto, CA 94301 (US)
(74) Representative: Dennemeyer & Associates S.A.
(86) International application number: PCT/IB2012/053685
(87) International publication number: WO 2013/011478

(56) References cited:
- WO-A2-2009/072115
- DE-A1- 10 240 725
- US-A- 5 700 252
- US-A1- 2009 266 365
- US-A1- 2010 071 693
- US-A1- 2010 242 967
- US-A1- 2010 280 626

## Description

### FIELD AND BACKGROUND OF THE INVENTION

The present invention relates to devices and methods for treating sleep disordered breathing and, more particularly, to devices which are designed for preventing collapse of airway tissue.

Snoring, excessive daytime somnolence, restless sleep, and obstructive sleep apnea (OSA) are manifestations of sleep-disordered breathing (SDB) which is characterized by abnormalities in respiratory patterns or the quantity of ventilation during sleep.

Obstructive sleep apnea, which is a common SDB is caused by sleep time collapse of airway tissue forming the pharyngeal wall, soft palate, epiglottis, and/or tongue.

Postural muscle tone is highest in wakefulness, decreased in non-REM sleep, and minimal or absent in REM sleep. Collapse of tissues experienced during REM sleep, leads to a cycle of air flow obstruction, disruption of sleep and arousal.

The recurrent sleep arousal and associated intermittent hypoxia and hypercapnia have been implicated in the occurrence of adverse cardiovascular outcomes. In addition, there is evolving evidence that SDB may contribute to insulin resistance and other components of the metabolic syndrome.

Numerous efforts have been made to treat sleep disordered breathing. These include uvulectomy, nasal reconstruction, adenotonsillectomy, uvulopalatopharyngoplasty (UPPP), genioglossal advancement as well as more complex surgical approaches such as maxillary-mandibular advancement, bimaxillary advancement, and tongue-base surgery.

The type of intervention selected for treatment is based largely on patient physiology and anatomy. A patient with a large uvula who snores and has few or no symptoms of apnea may benefit from uvulectomy or from the Pillar™ implant which is used to stiffen the soft palate.

Relief of nasal obstruction alone rarely cures OSA, however, patient tolerance and response to nasal CPAP are often improved, thus septoplasty, septorhinoplasty, and turbinate reduction may be indicated in patients who have predisposing anatomy.

Adenotonsillectomy is often performed in the pediatric population to correct loud snoring and restless sleep.

Uvulopalatopharyngoplasty (UPPP) is a procedure which is performed most often for treatment of OSA. This procedure includes tonsillectomy, reorientation of the anterior and posterior tonsillar pillars, and excision of the uvula and posterior rim of the soft palate.

Genioglossal advancement involves performing a mandibular osteotomy with anterior repositioning of the genioglossus-attached segment of the mandible. This procedure results in anterior displacement of the tongue.

Lingual tonsillectomy, lingualplasty, and laser midline glossectomy are procedures designed to reduce the mass of the tongue base. Temperature-controlled radiofrequency tissue ablation (TCRFTA) is used to reduce tissue mass of the tongue base and in the soft palate.

Despite considerable progress in our understanding of these disorders and the development of numerous treatment options, the principal therapeutic approach, continuous positive airway pressure (CPAP) which has been available since 1981 and exhibits poor patient tolerability and compliance remains the mainstay SDB treatment to this day.

There is thus a widely recognized need for, and it would be highly advantageous to have, an approach which is effective in treating sleep disordered breathing and yet is safe, minimally or non-invasive, reversible, and tolerable by patients.

Document US 2010/0242967 A1 discloses a nasal-pharyngeal bypass comprising a tube.

Document US 2010/0280626 A1 discloses a further device for treating sleep disordered breathing comprising a tube.

### SUMMARY OF THE INVENTION

The invention is defined by independent claim 1, further embodiments are defined by the dependent claims.

According to one aspect of the present invention there is provided a device for treating sleep disordered breathing comprising a device body designed for positioning within a pharyngeal lumen and being designed and configured for maintaining an open airway during involuntary closure of the pharyngeal lumen.

According to further features in preferred embodiments of the invention described below, the involuntary closure of the pharyngeal lumen is caused by collapse of airway tissue during sleep. the device body is configured as a cage of radially-spaced longitudinal ribs connected at tapering ends.

In a not claimed further example the device body is torpedo, football or banana-shaped.

According to still further features in the described preferred embodiments the device body is made from a porous mesh material.

According to still further features in the described preferred embodiments the device body has a porous surface and at least a part of it is axially curved to match the airway lumen with a radius of curvature of 40 mm or less, preferably about 20 mm.

According to the invention the device body is an open-ended tube with one or more curved regions to conform to the airway lumen with minimal contact with airway tissues.

According to the invention the curved region forms an arc of at least 30 degrees with a radius of curvature of 40 mm or less, preferably approximately 20 mm to match the curvature of the pharyngeal lumen along the resting soft palate.

According to the invention, the device body comprises a straight (distal) region and a curved (proximal) region with a proximal opening.

According to still further features in the described preferred embodiments, the angle formed by a vector normal to the proximal opening of the device body and the straight region is between 30 to 90 degrees.

According to a still further feature in the described preferred embodiments the device body is a thin-wall open-ended tube made from an elastomeric material 0.5mm or less in wall thickness with a curved top portion opening in the direction of the nasal cavity.

According to a still further feature in the described preferred embodiments the device body is an open-ended tube with at least some portion having a circular, oval or elliptical cross sectional shape.

According to still further features in the described preferred embodiments the device body is a crushable tube that can resist a maximum pressure of 100 grams (1 N) over a 1 cm length region before compressing to half width.

According to a still further feature in the described preferred embodiments the device body is made from an elastomeric material covered in a hydrogel or other hydrophilic coating that is either permanent, or alternatively degrades, dissolves or detaches over a preset time period.

According to still further features in the described preferred embodiments a patent airway is maintained during sleep.

According to still further features in the described preferred embodiments the airway muscles can collapse the device body during swallowing.

According to still further features in the described preferred embodiments the device body is reversibly and transiently compressed to a smaller cross sectional area during insertion and/or withdrawal of the device into or out of the nasal cavity.

According to still further features in the described preferred embodiments the device body is compressed by a water-soluble element.

According to still further features in the described preferred embodiments the device body is selected having a length of 3 to 8 cm and a diameter of 4 to 10 mm.

According to still further features in the described preferred embodiments the device body has a maximum surface area of 6,000 square millimeters, preferably 3,000 square millimeters.

According to still further features of the described preferred embodiments, the device body is a straight or curved tube with a dome or cone structure on its proximal end and a proximal opening positioned on a side wall of the tube.

According to still further features in the described preferred embodiments the device further comprises an anchoring element attached to the device body, the anchoring element being anchorable to or within a columella (tissue between the nostrils), a nostril, a nares, nasal cavity or nasopharyngeal tissue.

According to still further features in the described preferred embodiments the anchoring element is attached to the device body via a tether.

According to still further features in the described preferred embodiments the tether is selected having a length of 0.5-15 cm.

According to still further features in the described preferred embodiments, lateral bending forces of 10-100 grams would displace a 5 cm long section of the tether approximately 2-4 cm and the force required to cause a similar up and down displacement of the tether would preferably be 2-10 times as higher than those required for a lateral displacement.

According to still further features in the described preferred embodiments the device body is made from or coated with a material with a coefficient of friction of 0.5 or less when wet.

According to still further features in the described preferred embodiments the device body is coated by or fabricated from a material with a hardness of shore A 100 or softer, preferably 40 or softer and more preferably 20 or softer.

According to still further features in the described preferred embodiments the device body, anchoring element and tether are selected such that when the anchoring element is anchored to or within a nostril, nasal cavity or a nasopharyngeal tissue, the device body resides within a nasopharyngeal or oropharyngeal lumen.

According to another aspect of the present invention there is provided a device for treating sleep disordered breathing comprising a device body designed and configured for minimizing resistance to airflow through collapsed airway tissue while enabling sealing of the airway during swallowing.

According to yet another aspect of the present invention there is provided a method of treating sleep disordered breathing comprising (a) positioning a device within a pharyngeal lumen, the device being designed and configured for: (i) maintaining an airway or minimizing resistance to airflow during sleep; and (ii) enabling sealing of the airway during swallowing.

According to yet another aspect of the present invention there is provided system for treating sleep disordered breathing comprising the device of the present invention and delivering and positioning the device within a pharyngeal lumen through the nose.

According to yet another aspect of the present invention, the device body is a fully collapsible sheath with or without an internal stiffener that is inflated by an external air source and introduced into the nasal or oral cavity.

According to yet another aspect of the present invention, the device body extends down to or below the uvula.

According to yet another aspect of the present invention, the device body extends down to or below the base of the tongue.

According to yet another aspect of the present invention, the device body extends down to or below the epiglottis.

The present invention successfully addresses the shortcomings of the presently known configurations by providing a device capable of maintaining an open airway in collapsed airway tissue or minimizing resistance to airflow therein, especially during sleep and periods of loss of muscle tone.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
FIGs. 1A-B illustrate an MRI image of a transverse cross section at the level of the oropharynx showing an airway opening (black opening with arrow) in a collapsed state (FIG. 1A) and in an open state (FIG. 1B).
FIGs. 2A-B illustrate the relevant anatomy labeled in sagittal and posterior cut-away views.
FIG. 3 illustrates a further example of a device.
FIGs. 4A-B illustrate insertion of the device through the nose into position behind the soft palate overlaid on a sagittal view of the relevant anatomy.
FIG. 5 illustrates a body, tether and anchor of a further example of a device in its deployed configuration.
FIGs. 6A-B illustrates a further example with and without a water soluble element compressing the device body for easier insertion.
FIG. 7 is an endoscopic image showing the device illustrated in FIG. 4B positioned in the airway of a subject.
FIGs. 8A-B are sound traces from two nights during which the subject slept without (FIG. 8A) and with (FIG. 8B) the device.
FIGs. 9A-B illustrates an embodiment of the device of the present invention in a perspective view (FIG. 9A) and overlaid onto a sagittal section of the anatomy (FIG. 9B).
FIGs. 10A-B illustrates an embodiment of the device of the present invention in a perspective view (FIG. 10A) and overlaid onto a sagittal section of the anatomy (FIG. 10B).
FIGs. 11A-B illustrates an embodiment of the device of the present invention in a perspective view (FIG. 11A) and overlaid onto a sagittal section of the anatomy (FIG. 11B).
FIGs. 12A-B illustrates two embodiments of the present invention wherein device body is a straight (FIG. 12A) or curved (FIG. 12B) tube with a proximal end comprising a dome or cone structure and a proximal opening positioned on the side wall of the tube.
FIGs. 13A-C illustrates an embodiment of the device of the present invention in front, bottom, top and perspective views in the deployed state (FIG. 13A) and in the insertion state (FIG. 13B) with a removable stiffener inserted into the device body to make it roughly parallel to the tether. FIG. 13C illustrates how the nose clip anchor and stiffener are mounted on the columella between the two nostrils of a user.
FIGs. 14A-B illustrate a front, side, bottom and perspective views of two embodiments of the present nasopharyngeal airway design with FIG. 14A illustrating an opaque design and FIG. 14B illustrating a transparent design.
FIGs. 15A-D illustrate a device that does not have a curved upper portion placed in a nasopharnyx (FIG. 15A in schematic form and FIG. 15C as viewed using an endoscope), and which therefore impinges on sensitive airway tissues during swallowing causing discomfort (FIG. 15B in schematic form and FIG. 15D as viewed using an endoscope).
FIGs. 16A-D illustrate an embodiment of the present invention that has a curved upper portion placed in a nasopharnyx (FIG. 16A in schematic form and FIG. 16C as viewed using an endoscope) which therefore does not impinge on sensitive airway tissues during swallowing (FIG. 16B in schematic form and FIG. 16D as viewed using an endoscope) and is more comfortable than the embodiment in FIG. 15.
FIGs. 17A-C is an endoscopic image showing the internal lumen of the present invention before (FIG. 17A), part way through (FIG. 17B) and during (FIG. 17C) a swallow illustrating how swallowing forces are sufficient to collapse the device body of the present invention.
FIGs. 18A-B illustrates a further example comprising a highly collapsible or lay flat tube in the collapsed state (FIG. 18A) introduced into the nasal cavity and in the inflated state (FIG. 18B) when pressurized by an external air source.
FIGs. 19A-B illustrates a further example comprising a highly collapsible or lay flat tube in the collapsed state (FIG. 19A) introduced into the oral cavity and in the inflated state (FIG. 19B) when pressurized by an external air source.

### DESCRIPTION OF THE PREFERRED EMBODIMENTS

The present invention relates to a novel approach for treating sleep disordered breathing. Specifically, the present invention relates to devices and methods for minimizing resistance to airflow, or maintaining an open airway, through collapsed airway tissue while maintaining normal pharyngeal functions and being safe, reversible and highly tolerable for the patient.

The principles and operation of the present invention may be better understood with reference to the drawings and accompanying descriptions.

Before explaining at least one embodiment of the invention in detail, it is to be understood that the invention is not limited in its application to the details set forth in the following description or exemplified by the Example. The invention is capable of other embodiments or of being practiced or carried out in various ways. Also, it is to be understood that the phraseology and terminology employed herein is for the purpose of description and should not be regarded as limiting.

Although numerous approaches for treating sleep disordered breathing are presently available, none are as effective as the continuous positive airway pressure (CPAP) approach which remains the gold standard of treatment.

However, due to low patient acceptability and compliance, alternatives to CPAP are constantly sought after.

Effective treatment is hindered by several factors. Although the soft palate, tongue and pharyngeal walls have all been implicated as potential targets for treatment, the involvement of these tissues in the etiology of the disorder varies from one individual to the next. In addition, since these tissues are also involved in activities such as swallowing and talking, modification thereof can lead to discomfort or reduced function during waking hours. Furthermore, the airway tissues are extremely sensitive to touch and mechanical forces in general. The only things that these tissues normally contact are air, mucus and other airway tissues. As these tissues are quite dynamic, almost any foreign object coming into contact with them can cause significant discomfort unless properly designed and configured.

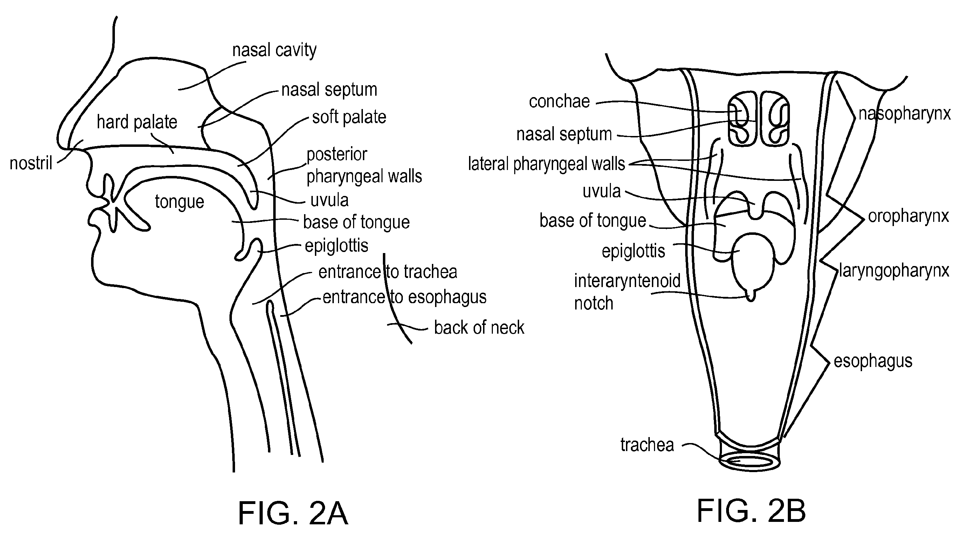

Figures 2a illustrates the relevant anatomy in a sagittal section while Figure 2b illustrates the same anatomy in a posterior cut-away view where the posterior pharyngeal wall has been sliced open along its midline and the pharynx and esophagus opened to expose the relevant tissues looking from the back of the head towards the front (relevant tissues are labeled).

While reducing the present invention to practice, the present inventor experimentally mapped out the most sensitive regions of the airway tissues. With reference to Figure 2a and 2b, these proved to be the nasal septum, the top region of the nasopharynx, the uvula, the top surface of the soft palate, the top and posterior pharyngeal wall, the tissue around the exit of the Eustachian tube, and all lymphoid tissues (adenoids, tonsils, etc). Contact with these tissues with anything hard or rough needs to be avoided in order to be tolerable.

Prior art nasopharyngeal airways deform and apply significant pressure on these sensitive tissues and are therefore intolerable for more than a short period. The present inventor realized that successful treatment of sleep disordered breathing can be achieved by maintaining a minimal airway opening at the region of airway closure and without applying excessive pressure to these sensitive airway tissue. Even a light force, applied on a small area, can cause levels of pressure (= force/area) that are intolerable. The challenge in designing a proper airway is to distribute the minimal force necessary to support the airway as uniformly as possible over the largest possible surface area while matching the softness and elasticity of the airway material to that of the airway tissue itself. Through extensive experimentation, various embodiments that meet these criteria were developed as part of the present invention.

During sleep disordered breathing, pharyngeal and palatal tissues collapse to substantially reduce the patency of the airway at the lower nasopharyngeal and oropharyngeal regions. In individuals suffering from sleep disordered breathing, such collapse can lead to complete closure of the airway and cessation of breathing.

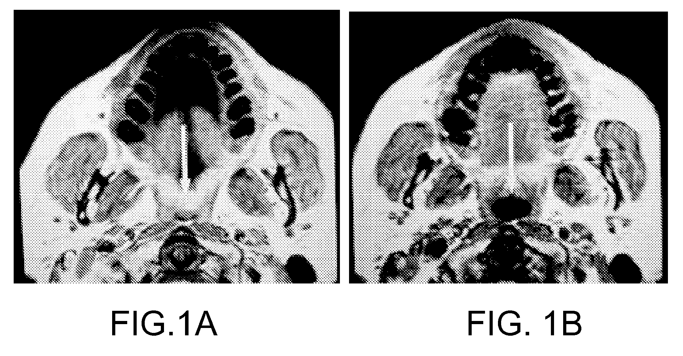

Studies have shown that airway muscle tissue loses tone intermittently when a person sleeps and that this loss of tone leads to collapse of this tissue and obstruction of the airway. As is illustrated in Figures 1a-b, collapse of pharyngeal tissues (including the lateral and posterior pharyngeal walls, the soft palate and the tongue) transforms the substantially round or oval airway opening into a narrow slit-like structure which is susceptible to further collapse and complete closure under the vacuum formed by a lung trying to expand. The narrow lumen forms a Bernoulli or venturi tube, which further reduces the air pressure at the constriction and strengthens the seal. Furthermore, since airway tissue is covered with sticky mucus secretions, the seal between the hydrophilic tissues becomes airtight and is only relieved when the person awakens due to lack of oxygen, thereby restoring tone to airway tissues and as a result opening the airway. These arousals prevent people suffering from this condition from sleeping properly.

Experiments have shown that nasal airway tubes having an internal diameter as small as 3 mm (JS Nahmias et al., Chest 1988; 94; 1142-1147) can help bypass the blockage formed by airway tissue collapse during sleep and restore ordered breathing, however such tubes are rigid and therefore are not tolerated for long-term placement in the airway. From this experiment one learns that a device which can maintain an opening through the collapsed airway sufficient for supporting ordered breathing would be capable of treating sleep disordered breathing in a subject suffering from, for example, snoring or sleep apnea. However these experiments have also shown that traditional nasal airway tubes are highly intolerable and as such were not used long term by most of the patients in the study, and are therefore not practical solutions to sleep disordered breathing.

As used herein, the phrase "airway tissue" refers to tissue which forms or surrounds the airway. Such tissue includes the tongue, the base of the tongue, the soft and hard palate, the uvula, the pharyngeal walls, pharyngeal mucosa, floor of the nasal cavity, nasal septum, nostrils, columella (the bridge of tissue between the nostrils) turbinates, exit of the eustachian tube, tonsils, adenoids and related lymphoid tissues, epiglotis and any surrounding tissues including, the tonsillar pillars, the levator and tensor palatini muscles, and numerous other muscles (hyoglossus, styloglossus, stylohyoid, stylopharyngeus, palatoglossus, palatopharyngeus and pharyngeal constrictors) that have varying functions.

Thus, according to one aspect of the present invention there is provided a device 30 for treating sleep disordered breathing in a subject, such as a human subject.

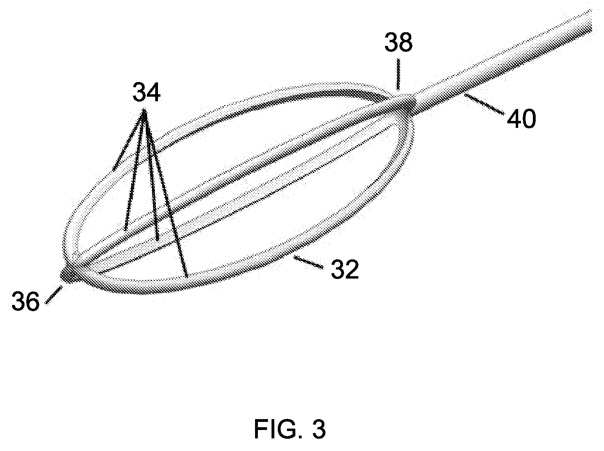

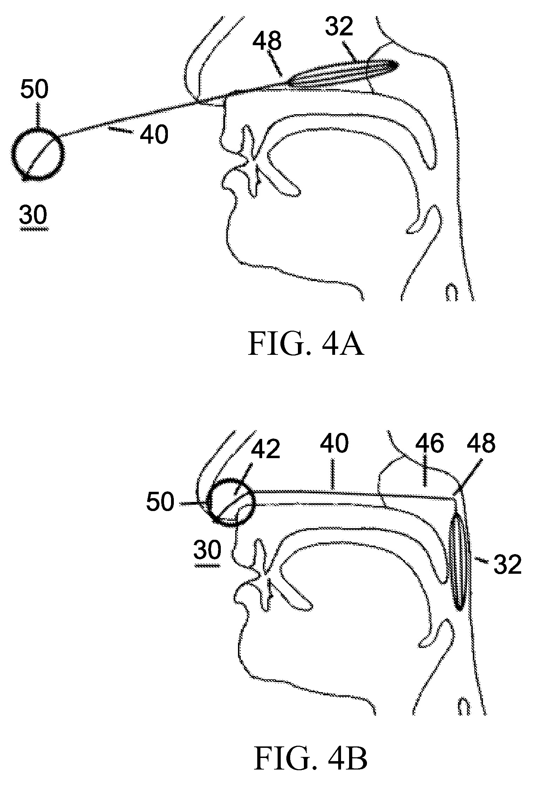

Figure 3 illustrates an example of a device body 32 of device 30 which is shaped like an elongated football or torpedo-shaped cage. Device body 32 is attached to tether 40. Device body 32 comprises one or more elliptically-shaped ribs 34 that are radially-separated and interconnected at distal end 36 and proximal end 38 (thus forming the torpedo or football shape of device body 32). Distal end 36 of device body 32 is shaped as a nose cone to minimize any sharp geometry from irritating the pharyngeal tissue and to enable easy insertion into the nasal cavity as will be described later. Proximal end 38 of device body 32 tapers and smoothly transitions into tether 40 to minimize mechanical irritation of airway tissue during device use and during device removal through the narrow nasal cavity opening. Tether 40 has a length suitable for anchoring device body 32 in position and includes a hinged portion 48 which functions to align device body 32 in the airway as is further described below with respect to Figures 4a-b.

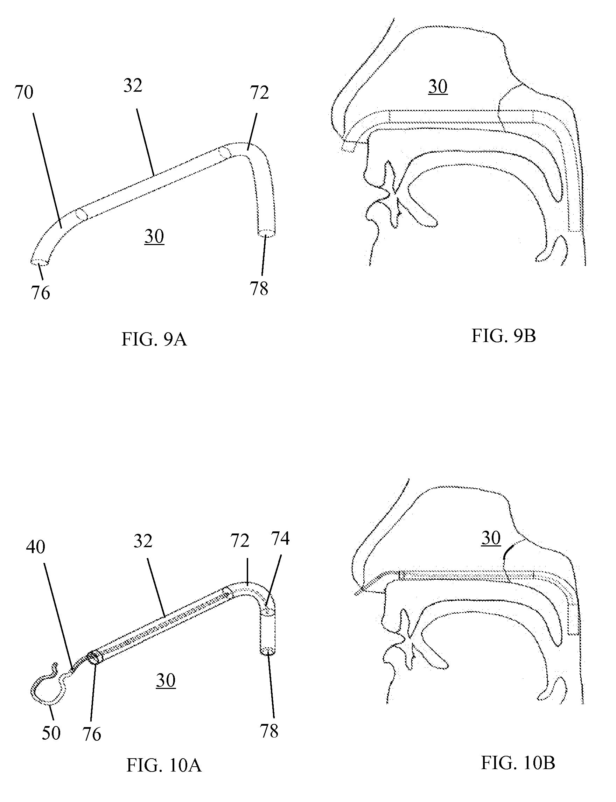

Alternatively, the long axis of device body 32, and ribs 34 or a mesh that makes up at least a portion of the surface of device body 32, can be curved (e.g. a banana shape or one of the shapes illustrated in the embodiments in figures 9-11 or 13) to match the shape of the relaxed airway lumen that would include a radius of curvature of 40 mm or less, preferably around 20 mm.

Ribs 34 are generally in line with the axis of the airway to minimize drag forces on device body 32 by the dynamic airway tissues, although they may be formed along a complex curve or spiral path from one apex to the other. A typical diameter each of ribs 34 can be 0.1-1.2 mm, preferably 0.3-0.8 mm, although the diameter, cross sectional shape and material composition of ribs 34 can vary over the course of their length to optimize their mechanical properties as far as bending, buckling and torsional stiffness. The length of device body 32 can be 1-6 cm, preferably 3-5 cm, in order to maintain patency across tissues contributing to airway blockage (from the top of the soft palate, through the lateral pharyngeal wall, uvula, base of tongue, and down to the epiglottis, see for example Rama, A.N., et. al, Chest 2002; 122; 1139-1147). In a non-collapsed state, the diameter of device body 32 at the midpoint between the tapered ends can be 2-12 mm, preferably 4-10 mm. This point of maximal diameter tapers towards the ends, where a diameter can be 1-4 mm. Such a configuration and size enables device body 32 to maintain a minimal airway when airway tissue is collapsed, yet ensures that device body 32 does not contact the entire airway lumen when the airway is open. Given that an open airway can be an oval with approximately 2-3 sq cm in cross sectional area during normal breathing, device 30 with a cross sectional area of 0.03 - 0.75 cm² is small enough to only tangentially touch an airway tissue and not interfere or apply forces on other parts of the airway during normal breathing. Furthermore, the maximal diameter of device body 32 should be between 2-12 mm to enable painless insertion, passage and removal through the nose and nasal cavity into and from the proper deployed position.

Ribs 34 can be made of one or more polymers (such as polypropylene, nylon, acetal, polycarbonate, PEEK, polysulfone and the like) and/or from a metal such as stainless steel or Nitinol. It has been experimentally determined that the surface finish of device body 32 (in all embodiments described herein) is critical to its tolerability during insertion into and deployment against airway tissues, which are extremely sensitive to rough and hard surfaces. At the cellular level, any rough points locally increase the pressure applied to the mechanoreceptors on the cells lining the airway tissues to intolerable levels. Therefore, ribs 34 can be constructed from any material provided that the outer surface of ribs 34 is soft and smooth. For example, ribs 34 can be constructed from nitinol wires which are then threaded through, dipped, painted, conformally or non-conformally coated or overmolded with a softer material such as silicone, styrene-butylene-styrene (SBS), polyurethane or a hydrogel (either natural such as gelatin, hyaluronic acid, collagen etc, or synthetic such as PEG or urethane based hydrogels at varying levels of reversible or irreversible crosslinking) to increase smoothness, softness, lubricity, and/or hydrophilicity of the tissue-contacting surfaces of ribs 34. Such coatings, which tend to have a shore A hardness of 100 or softer, can be erodible or dissolvable over time in the body, thereby exposing the underlying structural material of ribs 34 which can be of a different color or of an uncomfortable rougher surface finish, to make it clear to the user to not re-use the device beyond the number of applications intended. The coatings described above can be applied to all embodiments of device body 32 described in the present invention.

Collapse force of device body 32 can be controlled by the geometry of ribs 34 and the material from which they are made. A collapse force of 0.1 to 10 Newtons is desired. Collapse force is defined as the force applied to ribs 34 over their entire length until ribs 34 of device body 32 touch one another in their center portion. A collapsible configuration of device body 32 is preferred since it increases patient comfort, as the normal act of swallowing can still seal off the nasal cavity from the oropharynx. In the event that the sealing force does not collapse device body 32, airway tissue can still manage to seal by partially or fully invaginating around ribs 34. The number of ribs 34 in device body 32 can vary from 2 to 50, preferably 4 to 10. The space between each rib at the widest part of device body 32 is 0.5-8 mm, preferably 1-2 mm.

In yet a further example at least a portion of the long axis of device body 32 can be curved to match the shape of the airway lumen (e.g. banana shaped with a radius of curvature of 40 mm or less). At least a portion of device body 32 and be made out of a mesh with interwoven wire elements. Such a mesh can be made out of fine filaments of a polymer or metal such as stainless steel or nitinol and have an open area of 20-95%. The angle between interwoven filaments is chosen to allow for the mesh to be flexible and bend around a radius of curvature of 40 mm or less without buckling. The angle between interwoven filaments also allows device body 32 to expand or contract elastically in the radial and axial directions. The ends of the filaments can be combines into a closed tip forming distal end 38. Alternatively, the exposed ends of the filaments can be coated with a polymer to prevent them from contacting airway tissue.

In yet a further example device 30 has two or more device bodies 32 attached to a two-pronged tether 40 which are introduced through both nostrils. In this example the folding of tether 40 around the columella (tissue between the nostrils) acts as anchor 50.

Device body 32 or portions thereof can be wrapped in a thin outer membrane or partially or fully spirally wound with a circumferential element to prevent tissue from invaginating inside ribs 34. For example, the space between the two ribs 34 facing the uvula can be spanned by a membrane-type material (e.g. silicone or polyurethane) either bare or coated with materials and techniques as described for ribs 34 above. The membrane can be adhered to ribs 34 to form a suspended surface against which the uvula strikes when moving backward, instead of striking the posterior pharyngeal wall. Two more rearward ribs 34 keep this membrane surface and hence the uvula offset from the posterior pharyngeal wall thereby providing a patent airway between the membrane and the posterior pharyngeal wall. In the lateral direction, the ribs can act to prevent medial collapse of the lateral pharyngeal walls to also provide a patent airway, and the space between these two ribs can be left open or also covered with a membrane-type material. Note also that the distance between ribs can be greater in the lateral axis than in the anterior-posterior axis to better match the oval shape of a normal airway.

In a further example distal end 36 of device body 32 is a dissolvable "nose cone" or rib reinforcements that once dissolved in the body, partially loosens or completely frees ribs 34 from being connected to one another at distal end 36. Such an embodiment can make device body 32 more tolerable by reducing the mass of distal end 36 or can also prevent reuse of device 30 as device body 32 would be difficult to insert if ribs 34 are not connected together at distal end 36.

In a further example device body 32 or portions thereof are coated with a flavoring to allow a person to taste when the device is properly positioned. In a further example device 30 can be coated or impregnated with therapeutic agents for release into the airway tissues. For example, coating all or a portion of device 30 with an agent such as menthol can further open the airways and relieve nasal congestion or make the device more pleasant to use. In a further embodiment, device 30 or portions thereof can be covered with a lubricating (e.g. water based personal lubricant) and/or anesthetic agent (e.g. lidocaine), whether in gel, cream, powder or other form, that act to reduce discomfort during insertion.

Figures 4a-b illustrate the insertion and deployment of device 30 in a subject through the nasal cavity. Device 30 comprises device body 32, tether 40 with hinged portion 48 and anchor 50. In the insertion mode, hinged portion 48 is straight and device body 32 and rigid tether 40 are in line. The subject pushes device 30 into the nose preferably through a nostril that provides the easiest path through the nasal cavity (as predetermined by a doctor using endoscopic inspection or by inhaling vigorously through each nostril separately and choosing the side with the lesser resistance). Device 30 is pushed straight back parallel to the floor of the nasal cavity with device body 32 in front. If the space defined by the septum, nasal cavity floor and turbinates (conchae) is smaller than the diameter of device body 32, ribs 34 will momentarily compress under the force of insertion (or extraction) thereby facilitating introduction (or removal) of device 30. When device body 32 contacts the back of nasopharynx, it is forced downward, bending hinged portion 48 by up to 90 degrees and dropping into position behind the soft palate and/or base of the tongue.

Preferably, tether 40 is rigid enough to push on thereby facilitating insertion of device 30 without use of an applicator. Alternatively, tether 40 can be a tension member only (i.e. too flimsy to push on) and a detachable and more rigid applicator is used to push device body 32 into position, at which point tether 40 simply prevents further downward migration of device body 32 in the airway.

In a further example an applicator that moves in parallel to tether 40 can push on the distal end 36 of device body 32 so that during insertion, device body 32 elongates along its major axis and becomes narrower. When the applicator is removed, device body 32 returns to its natural shape in the airway.

It is important to position the device properly, as foreign objects below the uvula will be subject to strong downward forces due to the swallowing action of the base of the tongue. Foreign objects above the uvula are continually pushed up into the nasal cavity due to the lifting of the soft palate during swallowing. Since the airway blockage can occur above, at and/or below the uvula, device body 32 should cover these regions, but only to the extent necessary to span the obstruction. When properly positioned by tether 40, device body 32 tends to find a balance between these two opposing swallowing forces and can remain in position for as long as necessary without significant forces being applied to tether 40 or anchor 50.

With reference to Figure 4b, when device body 32 is in position, anchor 50 which is attached to the proximal portion of tether 40 is also in position against the external skin of the nostril. The distal portion of tether 40, termed herein as cantilevered portion 46, is suspended above the descending soft palate and keeps device body 32 in position and prevents device body 32 from moving out of position when the soft palate rises during a swallow. The proximal portion of tether 40, termed herein as angled portion 42, follows the contour of the proximal airway lumen and directs tether 40 out of the nostril with the minimum of tissue contact and pressure. In various embodiments, anchor 50 can attach to columella 51 between nostrils 53, or on outside of nostril 53. Anchor 50 can also be a ring of material too large to fit inside the narrowest portion of the nasal cavity to prevent device 30 from being swallowed or aspirated. Anchor 50 can rotate relative to tether 40 to enable attachment to either side of the external nostril or columella irrespective of which side of the nasal cavity device 30 in inserted into. Anchor 50 and tether 40 can be configured to apply controlled pressure on the nostril tissue or on the columella to keep device 30 in place. Alternatively, anchor 50 can have an adhesive, magnetic or other securing means for ensuring that device 30 doesn't move throughout the period of device use. Anchor 50 and tether 40 can be biased in such a way that tether 40 is forced against the floor of the nasal cavity which then minimizes the movement of device body 32 or acts to return it after a swallow where the soft palate tries to push device body 32 upwards and out of position.

Removal of device 30 is effected by simply pulling on anchor 50 to extract device body 32 from the nasal cavity, after which time it is either cleaned or disposed of.

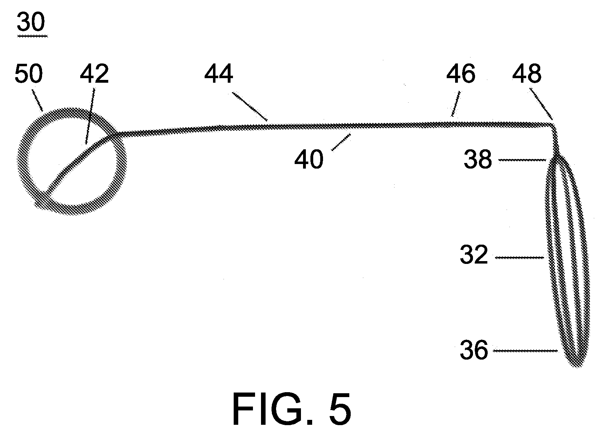

Figure 5 illustrates in more detail the features of device 30 in its deployed configuration. The length of tether 40 and dimensions of device body 32 can be varied based on the depth of the subject's nasal cavity and distance from the end of the hard palate to the epiglottis, which can be easily determined using a trans-nasal endoscopic examination, by using a trans-nasal sizing device or an external reference measurement, such as the distance between two anatomical features (e.g. the distance from the nostril to the ear opening). Tether 40 can vary in stiffness and cross sectional size along its length. For example, angled portion 42 can be thin and flexible to accommodate the curved exit from the nostril to anchor 50. Middle section 44 can be thin but stiff to run along or parallel to the floor of the nasal cavity without pressing on airway tissue which could cause nasal discharges as a foreign body response in the nasal cavity and to allow tether 40 to be used as the applicator to push device body 32 through the narrow nasal cavity. Middle section of tether 40 can be oval shaped so that it is tall in the vertical axis to stiffen the up and down bending moment of the tether, and thin in the horizontal axis to allow for easy bending laterally around the turbinates or deviated septums. Cantilevered portion 46 can be relatively thick and stiff as it is suspended in the lumen above the top of the soft palate behind the distal edge of the nasal septum (vomer). Hinged portion 48 can be thin and flexible (for example narrowed, drawn or formed injection-molded plastic or a hinge of elastic material) to allow for a 90 degree curvature between tether 40 and device body 32. The angles and dimensions of distal end 36 and proximal end 38 of device body 32 can be varied to minimize foreign body sensation in the airway and ease insertion and removal of device 30 through the nasal cavity. It has been experimentally determined that a minimum radius of 1 mm is required for distal end 36 of device body 32 to prevent it from getting stuck inside the nasal cavity during insertion, which causes discomfort. Radii larger than 1mm tend to self-center device body 32 in the biggest space available in the nasal cavity and make for a smooth and comfortable insertion.

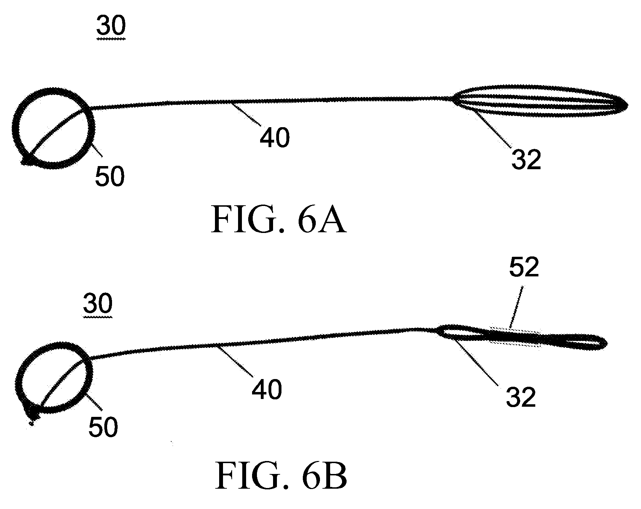

In a further example targeted at increasing ease of insertion, Figures 6a-b show device 30 in the insertion state without and with a compressive outer element respectively. In order to make device body 32 smaller in dimensions and easier to insert through the nasal cavity, a water soluble element 52 (for example polyvinyl alcohol-PVA) in the form of a spirally-wound string, bands, mesh, sleeve or sheath can be applied to device 30, device body 32, tether 40 or portions thereof to compress ribs 34 into a smaller diameter package, for example from 8 mm diameter deployed state to 2.5 mm in diameter insertion state for device body 32 as shown in Figure 6b. Once in the deployed state in the pharynx, water soluble band 52 dissolves in a matter of minutes and device body 32 returns to its original shape and size. In a further embodiment, ribs 34 are covered with a water soluble coating that allows them to be compressed, dried and therefore stuck together in the compressed state during insertion of device 30 and then to release from each other and expand upon dissolution of the coating in the humid pharynx. Other mechanisms for enlarging device body 32 from a small delivery state to a larger deployed state, such as applicator activated release mechanism or an over-tube that is withdrawn are also envisioned. Furthermore, enlarging or contracting device body 32 can be a reversible process activated by the user using a mechanism in the area of anchor 50 and that runs through or in parallel to tether 40 so that the subject never has to insert or withdraw device 32 in an enlarged state through the sensitive and narrow nasal cavity. For example, tether 40 and device body 32 can fit inside an overtube, sheath or hood (collectively referred to as an "outer tube") such that when tether 40 is retracted relative to the outer tube, device body 32 is compressed inside the outer tube. When tether 40 is pushed, device body is pushed out of the outer tube and resumes its deployed shape. Such a reversible change in size for device body 32 can range from an insertion/withdrawal diameter of 1-4 mm and a deployed diameter of 4-12 mm.

In one example device 30 can be self-introduced by the subject prior to sleep and removed upon waking in the morning. Many features can be incorporated into the design to enforce single or limited life use of device 30. For example, all or portions of tether 40 can be coated with water-soluble coating (with an outer coating of polyvinyl alcohol, gelatin or caboxymethylcellulose, polyvinylpyrrolidone, or polyurethane for example) that when dry stiffens tether 40 sufficiently to act as a compression member for introduction of device 30 into the nasal cavity, but then upon dissolving leaves all or portions of tether 40 too flimsy for pushing in a second time, but yet strong enough to act as a tension member to pull device 30 out of the nasal cavity after use. In a further embodiment, distal end 36 can be formed by a dissolvable nose cone that allows for a single insertion with the proper radius, and thereafter the radius of distal end 36 is such that it becomes unpleasant or difficult to insert.

Device 30, portions thereof, or coatings applied thereto can dissolve or detach from the underlying substrate material in an aqueous environment over time (for example coatings made of a hydrogel) to degrade and disappear following their intended service time. Since the in vivo degradation or detachment time of such materials is well known in the art, design of device 30 or portions thereof capable of such timed degradation or detachment is well within the capabilities of the ordinary skilled artisan.

Device 30, or portions thereof in all examples herein, can have a non-stick, hydrophobic or low coefficient of friction surface of 0.5 or less (for example made from acetal with a coefficient of friction of 0.25 or silicone which is highly hydrophobic) thereby ensuring that mucus secretions do not accumulate on or within the device and that peristaltic forces on device body 32 are minimized due to its low friction and low surface area within the pharyngeal lumen.

Alternatively, device 30, or portions thereof in all examples herein, can have a lubricious hydrophilic coating applied to it so that the coefficient of friction is also less than 0.5 when wet. Device body 32 can combine a hydrophobic interior surface to prevent clogging with a hydrophilic outer surface to increase comfort and biocompatibility.

Since in the example illustrated in Figure 3, device body 32 is open in all directions, it cannot clog due to mucus or other discharges. By way of example, the outer envelope defined by device body 32 in Figure 3 is only around 10% filled with ribs 34, with 90% of the circumference remaining open. This ratio can be reduced to below 50% filled and 50% open even if device body 32 is formed by a fine mesh and the un-cloggable aspects still applies since air can enter the sides of device body 32, and not just through the top and bottom openings of a closed-wall tube.

Device body 32 is designed such that it resists a crush force of .01 - 20 Newtons, or preferably 1 - 10 Newtons per 4 cm length thereby being capable of supporting the pharyngeal walls against collapse during sleep and yet at least some portion of the device body flattens (crushes) and seals when greater pressure is applied thereto, by for example, pharyngeal wall pressure applied during swallowing, eating, speaking, coughing etc. More preferably device body, which could be 5-10 mm in diameter when open, flattens completely to be at most 1.5 mm thick when a maximum of 100 gram mass (= 1 N force) is applied to a 1 cm long segment of the device body. This enables introduction without discomfort through the narrow slit-like passageway between the turbinates and nasal septum which, can be only 1-3 mm wide.

The numbers above are derived from the universal efficacy of 10 cm of H₂O as delivered by modem CPAP machines which form an "air splint" that opens collapsed airways, mainly in the lateral direction, but also in the anterior posterior direction. 10 cm of H₂O in a 4 cm long section of an airway 8 mm in diameter converts to a radial outward force of 0.3 Newtons. Device body 32 is configured to provide similar uniform pressures on the collapsing airway tissues. For example, a 4 cm long by 8 mm diameter device body 32 with four 0.6 mm diameter ribs 34 made of polypropylene (G70 from Carmel Olefins, Israel) can resist a crush force of approximately 1 Newton. The same device body 32 made from a softer grade of polypropylene (R50 from Carmel Olefins, Israel) can resist a crush force of approximately 0.5 Newtons. Such device bodies are sufficiently stiff to provide a patent airway yet soft enough to not irritate sensitive airway tissue, and collapsible enough to form a seal that provides velopharyngeal sufficiency during swallowing, talking and coughing. Finding the light balance between proper stiffness and collapsibility is key to the functioning and tolerability of the present device.

Because device body 32 is suspended in the airway and is substantially smaller than the diameter of the airway, the force that device body 32 applies to the airway tissues is effectively zero during steady state breathing, which makes device 30 highly tolerable. Only during talking, coughing, swallowing and SDB does the airway tissue try to occlude the lumen and then device body 32 comes into substantial contact with the airway tissues, at which point ribs 34 exert a radially-outward directed force proportional to the displacement of rib 34 inwards. Ribs 34 can collapse by bending inward towards an imaginary centerline running through the apices of device body 32, and/or torsionally deform and bend sideways. The greater the collapse of the airway tissue, the greater the displacement and hence the counter force applied by ribs 34, so that the maximum forces are only applied in an outward radial direction as the airway tries to fully collapse. This design ensures maximum tolerability by minimizing tissue contact and minimizing forces applied to the tissue during the majority of the time when no airway support is required. The maximum forces are applied by device 32 only as and when needed to a minimum amount of tissue (as defined by the surface area of device body 32) during episodes of SDB.

In a further example device body 32 can be made from a self expanding nitinol material which is compressed under cold water (~ 25 °C) and expands at body temperature. Alternatively, ribs 34 can be collapsed into a thin and low profile device body 34. Linear pushing or retraction of a central element in device body 32 can push out ribs 34 to their deployed state. Deployment can occurs manually or dynamically in response to a SDB-related signal (e.g. snoring sound, airflow, air pressure, tissue pressure, vibration sensor, mattress motion, motion sensors, peripheral arterial tone sensors, EEG sensors, etc). The power source and control unit can be outside body in the nose clip region. Deployment of ribs 34 can be done under closed loop control with ribs 34 expanding up to pre-defined limits just until the SDB (e.g. snoring) stops. This allows actuation just to the extent necessary to eliminate the SDB and just during the SDB episodes when the user is asleep. When awake or sleeping without SDB, the device would not be deployed and therefore not felt in any appreciable manner.

Other deployable elements are envisioned in the embodiment above, such as arms that extend from upper pivot point in shape of a flower without distal apex. When collapsed, they form a single element. Alternatively, the deployable elements are gas or liquid inflatable arms or inflatable projections which prod open the airway on demand.

According to the present invention, device 30 (illustrated in Figure 9a in a perspective view and in Figure 9b overlaid on a sagittal section of the relevant anatomy), is configured as a thin-walled tube-shaped device body 32 with proximal opening 76 and distal opening 78. The device body 32 is pre-shaped to follow the natural anatomy of the airway with minimal contact with airway tissue when proximal opening 76 is positioned at the entrance to the nares. As such, device body includes a first region which is pre-bent at roughly 45 degrees in region 70 to accommodate the angle between the nares and the entrance to the nasal cavity and pre-bent again at roughly 90 degrees (range 30 - 110 degrees) in region 72 to accommodate the angle between the floor of the nasal cavity and the posterior wall of the nasopharynx (i.e. the "elbow" region connecting the horizontal floor of the nasal cavity to the vertical upper nasopharynx).

Such pre-shaping ensures that device body 32 does not apply any substantial pressure on any region of the airway from the nostrils to the region surrounding distal opening 78 of device body. As is mentioned hereinabove, airway tissue is extremely sensitive to contact pressure applied by foreign bodies as normally the only materials they are in contact with are air, mucus secretions and other airway tissues. Thus, for operability, a device not only needs to provide an open airway during tissue collapse but it must also be tolerated by the user over extended periods of time.

Although use of tether 40 and to a lesser degree anchor 50 is presently preferred, it will be appreciated that the configuration of device body 32 also allows for self-anchoring. As is illustrated in Figure 9a-b, device body 32 can be held in position by the friction between device body 32 and airway tissue alone. If such a configuration of device 30 is accidentally forced deeper into the nasal cavity during application, device body 32 could potentially move down through the nasopharynx. However, in normal use such movement is unlikely due to the fact that distal opening 78 of device body 32 resides above the epiglottis, and since device body 32 is easily crushable and thus is not easily grabbed by peristaltic swallowing forces. Optionally, device body 32 could be flared outwardly in the area of the nares to prevent proximal end 76 from entering the nasal cavity. In addition, device 30 can be more discrete when in use since proximal opening 76 of device body 32, or a pull tab connected thereto (not shown) positioned at or in the nares is enough to allow a user to remove device 30 from the nasal cavity.

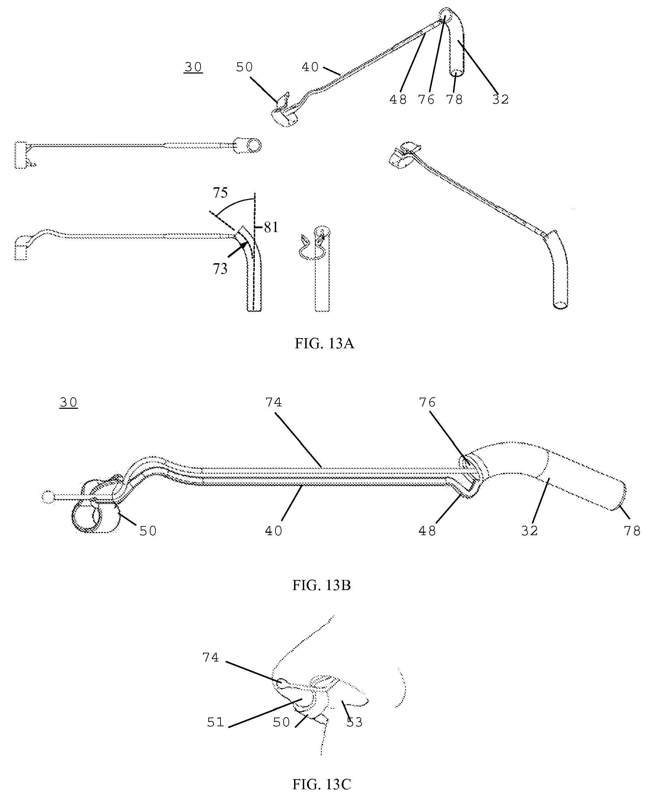

Device body 32 of this embodiment of the present invention is flimsy and collapses easily under axial, lateral or radial loads. Therefore, the use of a stiffener element 74 (not shown) may be utilized to allow device 32 to be self-inserted. Stiffener element 74 can be thin (e.g. 0.5 - 1.5 mm diameter rod of any common thermoplastic material) and can be attached to the inside or outside surface of device body 32, or be positioned freely (unattached to device body 32) in the lumen of device body 32. Stiffener element 74 can be straight or also pre-bent to partially or fully follow the shape of device body 32. Stiffener element 74 can be attached to device body 32 at one or more points (e.g. the proximal or distal edge of device body 32) or it can be fully removable from device body 32 after introduction into the nasal cavity, in which case, removal of stiffener element 74 can be effected with a feature that extends from device body 32 out of the nostril(s), as illustrated in Figure 13c.

Figures 10a-b illustrate yet another embodiment of device 30 in a perspective view (Figure 10a) and overlaid on a sagittal section of the relevant anatomy (Figure 10b). Device 30 is a thin-walled tube-shaped device body 32 with proximal opening 76 and distal opening 78. In this embodiment, proximal opening 76 resides at the entrance to the nasal cavity and is pre bent at roughly 90 degrees (+/- 20 degrees) in region 72 to accommodate the angle between the floor of the nasal cavity and the rear wall of the nasopharynx. Anchor 50 connects tether 40 to device body 32. Stiffener element 74 can be used in this embodiment as well and can be a simple extension of tether 40. In this embodiment of device 30, contact between device body 32 and the inner sensitive surface of the nares is avoided, and thus use of this device 30 can be comfortable and discreet.

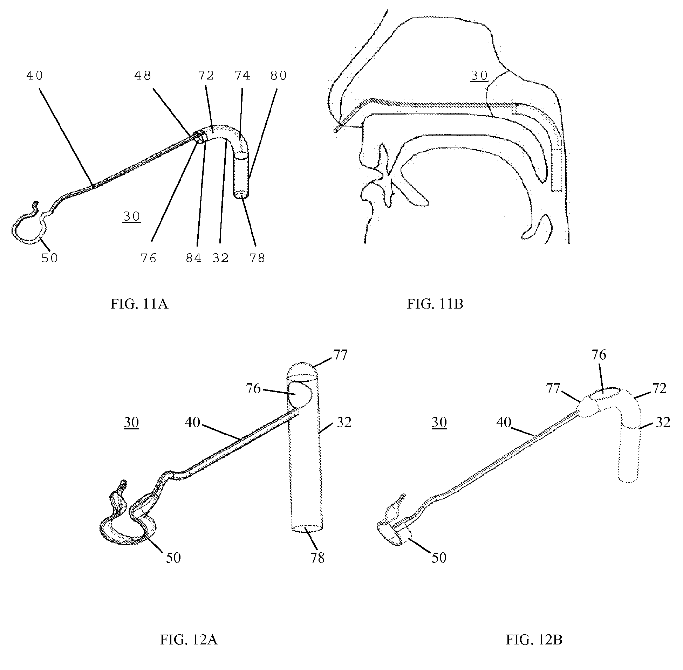

In one presently preferred embodiment of the present invention, Figures 11a-b illustrate device 30 in a perspective view (Figure 11a) and overlaid on a sagittal section of the relevant anatomy (Figure 11b). Device 30 of this embodiment is a thin-walled tube-shaped device body 32 with proximal opening 76 and distal opening 78. In this embodiment, proximal opening 76 is at the distal end of the hard palate (distal to the turbinates and/or distal edge of the nasal septum) and is pre bent at 90 degrees in Figure 11b (though a range of 30-120 degrees is also possible) in region 72 to accommodate the angle between the floor of the nasal cavity and the rear wall of the nasopharynx (i.e. it follows the elbow region described above). Anchor 50 connects tether 40 to device body 32, while stiffener element 74 can be a simple extension of tether 40 or a separate element running roughly parallel to tether 40 with the distal end of stiffener 74 extending into device body 32. Stiffener 74 can be curved to match the curve of device body 32, or stiffener 74 can be straight and as a result be inserted into and pivot straight portion 80 device body 32 to be roughly parallel (+/- 30 degrees) to the axis formed by tether 40 to facilitate easier entrance of device 32 into the nasal cavity as illustrated in Figure 13b. In this embodiment, stiffener 74 curves when device body 32 engages the posterior pharyngeal wall. Stiffener 74 can stay in device body 34 during use of the device, or it can be withdrawn after proper positioning of device body 34 in the pharynx.

In this embodiment of Figures 11a-b, contact between device body 32 and the sensitive airway tissues of the septum and turbinates is avoided, and the air entering into device body 32 is properly warmed and humidified by the turbinates as occurs in normal nasal breathing. Furthermore, in the case of congestion on the side of the nasal cavity through which tether 40 passes, air from the opposite side of the nasal cavity can still enter device body 32 through proximal opening 76 as it rests behind the nasal septum separating the two halves of the nasal cavity. Additionally, moving portions of the airway tissues, namely the soft palate and lateral pharyngeal walls, and other sensitive regions of the airway tissue such as the top of the nasopharynx and posterior pharyngeal walls, are not subjected to any appreciable contact forces by lumen-conforming device body 32, proximal opening 76, and tether 40. When such contact does occur, it is against the smooth and well-curved surface of device body 32 that minimizes force concentration on the tissue due to the rim of proximal opening 76, sharp edges, kinks or surface irregularities. Device body 32 in Figures 11a-b has two regions, straight region 80 and elbow-shaped bent region 72. With reference to Figure 13a, angle 75 formed by a vector normal to proximal opening 76 and axis 81 defining straight portion 80 can be in the range of 30 to 90 degrees. The purpose of region 72 is to prevent the edge of proximal opening 76 from contacting the very sensitive rear or top of the nasopharynx. Furthermore, proximal opening 76 can include a more crush-resistant reinforced or geometrically thicker region 84 that prevents proximal opening 76 from collapsing due to the vacuum forces generated in device body 32 during inhalation since the collapse of a flimsy and uniform tube under vacuum will tend to first collapse proximal opening 76.

By way of example, it has been experimentally determined that if device body has a 6.4 mm outer diameter with a wall thickness of 0.25 mm made out of Shore A 20 silicone, then region 84 can be a ring region of 0.8 mm wall thickness (+/- 0.4 mm) by 1.5 mm wide (+/- 1 mm) in order to prevent device body 32 from collapsing during inhalation when being used to breathe through like a straw. Region 84 should not be too rigid as it still needs to collapse in order to be delivered through the nasal cavity without causing discomfort. The dimensions above achieve both goals of resisting device body 32 collapse due to the vacuum formed by inhalation together with tolerability during insertion.

It will be appreciated that the embodiments of Figures 9a-11b can also be configured such that proximal opening 76 and distal opening 78 reach other positions to those described above, including having proximal opening 76 extend into the nasal cavity proximal to the vomer (end of the nasal septum) and distal opening 78 extend up to or beyond the epiglottis to provide an airway behind the base of the tongue.

Existing nasal airways tend to be only slightly curved (maximum 45 degree angle between both ends in a smooth curve with a radius of curvature of approximately 200 mm over an approximately 15 cm distance) and relatively stiff to enable easy insertion into the nasal cavity as part of a medical procedure. Samples materials for such airways include hard plastics like PVC. A 6 mm ID airway might have 1 mm thick walls and be highly resistant to crush and bending forces.

Therefore, with reference to Figure 13a, prior art tubes would be nearly impossible to introduce if they were pre-bent in an arc of 30 to 90 degrees in region 72 with radius of curvature 73 of 40 mm or less, or preferably approximately 20 mm, since they would be very hard to advance along the straight and narrow floor of the nasal cavity. Therefore, as a design compromise between comfort and ease of insertion, prior art nasal airways are curved only gently and are then forced to conform to the 90 degree direction change in the airway lumen by forces exerted through contact with the sensitive airway tissue in the back of the nasopharynx. Such forced curving causes significant discomfort and even retropharyngeal ulcers. Furthermore, the space between the turbinates and the nasal septum is usually just a few millimeters wide at most, and existing airways are 5-8 mm in outside diameter and not crushable, and as such, prior art configurations also displace and apply pressure to the sensitive septum and turbinates, causing further discomfort.

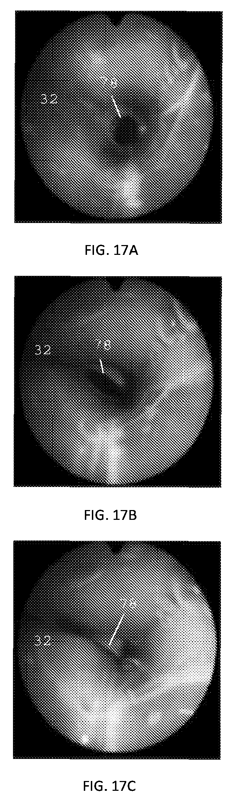

In sharp contrast, the device of the present invention in its resting state conforms to the nasal airway lumen with little to no force applied to the surrounding airway tissues. Device body 32 is preferably fabricated from a material of Shore A hardness value of 100 or less, preferably Shore A of 40 or less and more preferably Shore A of 20 or less, with a wall thicknesses of 0.10 - 0.50 mm, or preferably 0.2 to 0.4 mm. For example, it has been experimentally determined that a 6.4 mm outer diameter tube of 4 cm in length (as illustrated in Figure 13) with a wall thickness of 0.25 mm made out of silicone with Shore A hardness of 20 will crush half way (becoming 3.2 mm wide) under a force of approximately 100 grams (IN) per 1 cm length of tubing. Such a tube, if reinforced to 0.8 mm wall thickness at region 84, can be breathed through to provide sufficient air for a sleeping user indefinitely without collapsing due to vacuum forces created by the moving air though device body 32, yet will crush under the forces of swallowing or speaking. Figures 17a-c show an endoscopic view from within the top portion of device body 32 as illustrated in Figure 13a-c and as constructed and deployed in Example 2 which follows, before (Figure 17a), part way through (Figure 17b) and during the maximum force applied by a swallow (Figure 17c). Figure 17c clearly shows that device body 32 fully collapses between proximal opening 76 (where the endoscope is positioned) and distal opening 78 due to swallowing forces.

Device body 32 configuration described above achieves the goal of providing sufficient airway support but yet being tolerable by not resisting crushing due to insertion, swallowing or speaking forces. Another combination that achieves the same goal is device body 32 configured as above but with a wall thickness of 0.35 mm made from silicone with a Shore A hardness of 10. This makes device body 32 very crushable for easy insertion between the septum and turbinates. Additionally, as verified endoscopically and when viewed in a coronal section, the cross sectional shape of a normally circular device body 32 in the nasal cavity is deformed to be an oval 1 to 3 mm wide with a longer vertical axis than a lateral axis to conform to the 1 to 3 mm wide vertically oriented airway lumen between the turbinates and the septum. This deformation applies less than 10 N of force per cm on these sensitive tissues during insertion and use, therefore avoiding any damage to the tissue or discomfort. As is shown in the Examples section which follows, and in sharp contrast to prior art nasopharyngeal airways, insertion and use of the present device in subjects resulted in little or no discomfort over extended periods of time.

Given that in general, a thin walled tube cannot bend without buckling (as opposed to a thick walled tube such as the prior art nasopharyngeal tubes), the thin tube of device body 32 needs to be pre-formed in the proper curvature to follow the lumen defined by the airway tissues. Otherwise, a gently curved thin walled tube will buckle when forced to conform to the 90 degree bend in the airway at the back of the nasal cavity, thereby fully or partially sealing its internal lumen. A thin walled tube can only bend +/- 20 degrees or so before collapsing on itself, thereby sealing its internal lumen. In the embodiments illustrated in Figures 9a-14, and in contrast to prior art nasal airways which are crush and kink resistant, portions of device body 32 of the present invention are designed to intentionally elastically and reversibly kink, deform, bend, crush and/or buckle during insertion of device 30 to accommodate the narrow geometry of the nasal cavity (but only to the extent permitted by stiffening element 74). Once reaching its intended position, device body 32 elastically returns to its natural shape that conforms to the airway lumen and defines an open and continuous internal lumen of the same size and shape as that of device body 32 when outside the body until acted on by swallowing forces. Therefore, and in contrast to prior art nasal airways, the shape of device body 32 outside the body (Figures 9a, 10a, 11a) is identical to that of a device body 32 when positioned inside the relaxed airway (Figures 9b, 10b and 11b). Prior art nasophayngeal tubes are more curved in the airway than they are outside the body. As is shown in the Examples section which follows, experiments have shown that temporary deformations during device insertion do not create any discomfort to the user since the underlying material forming device body 32 is easily crushable, smooth, soft, lubricious and elastic.

In a further embodiment illustrated in Figures 12a-b, device body 32 is a thin-shelled (e.g. 0.1 - 0.5 mm wall thickness) straight (Figure 12a) or curved (Figure 12b) tube with proximal end comprising dome 77 or cone structure connected to tether 40 and proximal opening 76 positioned on the side wall of device body 32. Proximal opening 76 can be in the shape of a circle, ellipse, oval, square or any other polygonal shape and can be reinforced against collapse by region 84 as describe elsewhere. Device body 32 can have one or more proximal openings 76 placed at any radial position along the circumference of device body 32 such as facing up, to the side or down.

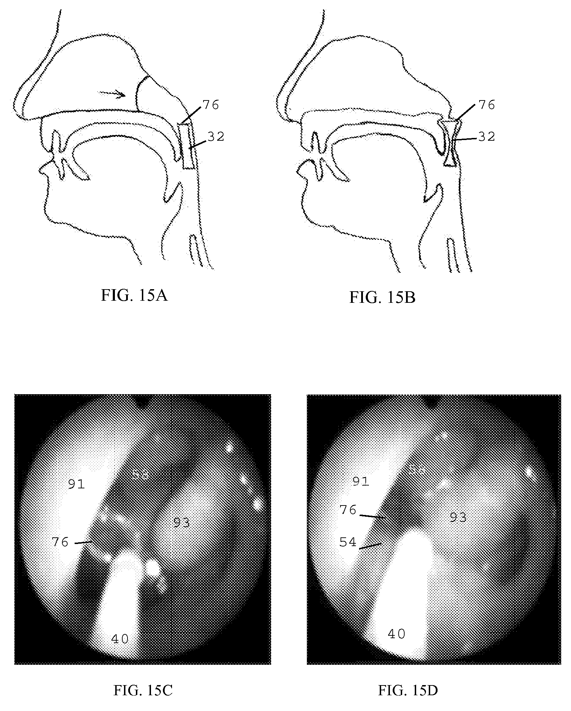

Dome 77 serves to prevent any exposed edges or rims, such as the rim of proximal opening 76 from touching or impinging sensitive airway tissues (as illustrated in Figures 15b and 15d). Contact of dome 77 is highly tolerable, as dome 77 is soft and collapsible, and there are no sharp edges or corners to cause local pressure peaks. In this embodiment, the angle defined by a vector normal to proximal opening 76 relative to the axis of device body 32 close to distal opening 78 can be anywhere in the range of 90 degrees (Figure 12a) to 0 degrees (Figure 12b).

Generally, it is desired to keep proximal opening 76 suspended within the airway lumen and not touching airway tissues in a relaxed airway, not only to prevent discomfort, but also to minimize the entrance of mucus secretions into proximal opening 76 which can clog device body 32.

Device 30 or portions thereof in the embodiments of Figures 9a-14 can be coated with a lubricious hydrophilic coating (e.g. coatings made by Surmodics Inc, Hydromer Inc. Biocoat Inc. etc). The extent of attachment or crosslinking of the coating can be varied to enable the coating to adhere indefinitely, or alternatively dissolve, degrade or detach after a set period of time, (e.g. at least half of the coating is no longer functional after 12 hours in the pharynx of a user). This intentional degradation of coating effectiveness would make it less likely to reuse device 30 if it is designed as a single use product, especially if a less suitable or different colored surface is exposed after the elimination of the coating.

Although device body 32 of Figures 9a-14 is preferably fabricated from a single unitary tube, other configurations are also envisaged herein. For example, device body 32 can be formed with a single distal opening 78 and two proximal openings 76 that branch out in a Y shape. The Y portion can rest on the back of the vomer (distal end of the nasal septum) and each proximal opening 76 can extend proximally into each of the nasal cavities on both sides of the septum. Alternatively, the vomer itself can form the septum separating both sides of the Y shaped device body 32. This configuration enables a definite landmark to be used for positioning and/or anchoring device body 32 in the pharynx (for example, the Y should rest on or immediately distal to the edge of the vomer). In this embodiment, tether 40 can be axially elastic to allow for overextension of device body 32 into the nasopharynx using removable stiffener element 74. Upon withdrawal of stiffener element 74, the Y portion of device body 32 retracts and seats against the distal edge of the vomer.

In a further embodiment, device 30 can include a plurality (e.g. 2-10) of device bodies 32 made out of thin walled small diameter tubes (e.g. 1-3 mm OD with 0.1-0.2 mm thick walls) that can successfully bend 90 degrees with a radius of curvature of 40 mm of less, preferably approximately 20 mm which is the curvature along the top of the relaxed soft palate, without kinking. The cumulative cross sectional area of multiple such tubes can provide sufficient open area for normal breathing. Furthermore, multiple little tubes can orient themselves more easily within the airway lumen without impinging on airway tissue relative to a larger diameter tube. Multiple device bodies 32 can be merged into one large device body 32 at some location along device 30.

In an alternative embodiment illustrated in Figure 13a-c, device body 32 is designed so that straight region 80 has a 90 degree (+/- 30 degree) angle in its resting state relative to the axis defined by tether 40. Tether 40 has a hinged portion 48 (e.g. silicone of around 1-3 mm in diameter) so that device body 32 can pivot about tether 40 accommodate any angle. Stiffener element 74 forces device body 32 to maintain straight region 80 to be at an angle of between 150 to 180 degrees relative to tether 40 by pivoting device body 32 in the direction of the axis formed by tether 40, potentially also deforming device body 32 slightly in its resting state outside the body. The deformation can narrow or fully occlude the inner lumen of device body 32 with no negative effects. This makes it easier to insert device body 32 into the nasal cavity as it forms a straighter axis than the 90 (+/-30) degree of the deployed state in the pharynx. When the leading edge of stiffener element 74 reaches the top of the nasopharynx, it is forced to bend roughly 90 degrees down along the back of the nasopharynx. In so doing, it restores the angle of straight region 80 to 90 (+/-30) degrees relative to tether 40, thereby fully opening the inner lumen of device body 32, while at the same time forcing device body 32 to rest against the back of the nasopharnyx with a force of around 0-15 grams (0 - 0.15 Newtons). Such a force is far less than the force required to bend a prior art nasopharyngeal airway to the 90 degrees required to conform to the airway, and hence is significantly more tolerable.

By having device body 32 biased against the back of the nasopharynx with up to 15 grams of force, it has been experimentally determined that device body 32 is more stably positioned to resist movement during swallowing (mainly the soft palate rising and pushing device body 32 up and tether 40 out and in the process aggravating sensitive airway tissue). Also, by being biased back and away from the uvula, device body 32 is less likely to touch or stimulate the uvula which avoids a gag reflex and minimizes the foreign body sensation in the nasopharnynx.

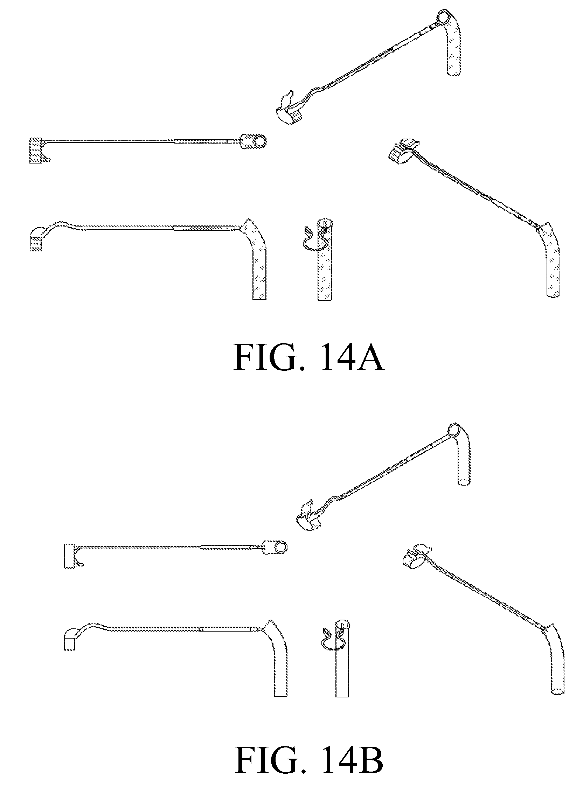

Figures 14a-b illustrate a front, side, bottom and perspective views of two embodiments of the present nasopharyngeal airway design with Figure 14a illustrating an opaque design and Figure 14b illustrating a transparent design.

In yet a further embodiment, device body 32 can have variable cross sectional areas or shapes along its axis from its proximal opening 76 to its distal opening 78. For example, proximal opening 76 of device body 32 can be a circle to best resist crushing due to the vacuum formed in device body 32 during breathing. Distal opening 78 of device body 32 can be slit like or oval shaped to better conform to the oval shaped airway (when viewed from above - see Figure 1b). Example dimensions are a circular cross section of 4 - 10 mm in diameter at proximal opening 76 gradually transitioning to an elliptical cross section of 8 - 12 mm long axis and 2 - 4 mm short axis at distal opening 78 behind the uvula or the base of the tongue. Transition between the shapes of the openings can be abrupt or gradual. By keeping the distal opening 78 of device body 32 as flat as possible, there is a broader width to act as a target for the uvula to strike against (instead of the posterior pharyngeal wall) and less of a projecting object that may create a sense of swallowing a foreign body behind the base of the tongue. Alternatively, the hardness or wall thickness of device body 32 can vary along its length. For example, region 84 near the proximal opening 76 of device body 32 can be thicker than the other regions of device body 32 to counter crushing of proximal opening 76 due to the vacuum formed in that area during breathing. Another example is that an elliptical cross section can be thicker than a circular cross section to resist collapse along the narrow axis. Alternatively, device body 32 can have one or more internal ribs or other internal supports to prevent full collapse of device body 32.

In yet a further embodiment, device body 32 can be extended by up to 2 cm beyond distal opening 78 as a flat flange to act as a "shoe horn" to separate the airway tissues. In yet a further embodiment, the plane defined by proximal opening 76 and/or distal opening 78 can be tapered or angled such that they are anywhere from perpendicular to parallel to each other.

In yet a further embodiment, tether 40 can have hinge portion 48 just proximal to proximal opening 76 of device body 32 which allows a controlled force to keep device body 32 at a specific angle relative to tether 40. By way of example, the resting state of hinged portion 48 can maintain the angle between straight region 80 and tether 40 of device body 32 to be in the range of 110-180 degrees, allowing for easy horizontal insertion along the floor of the nasal cavity. Upon reaching the top of the nasopharynx, straight region 80 of device body 32 turns down along the vertical back of the nasopharynx and assumes a roughly 90 degree angle relative to tether 40. The change of angle is accommodated by hinged portion 48 (e.g. an elastic or plastic bending of a region of tether 40) that applies a controlled force (e.g. 15 grams or less) when bent. Allowing a change of angle between device body 32 and tether 40 without compromising the integrity of the inner lumen of device body 32 is useful in accommodating varying anatomies and the dynamics of chin up and chin down head movement which change the angle defined by the floor of the nasal cavity and the back of the nasopharynx.

The connection of tether 40 to device body 32 (either with or without hinge portion 48) can be at the center of proximal opening 76 or at any hour such as the 6, 3, 9 or 12 O'clock position along the circumference of proximal opening 76. An advantage of connecting tether 40 to device body at the 3 or 9 O'clock position is that device body 32 can thus be centered in the pharynx since tether 40 emerges either to the light or left of the nasal septum. Device body 32 is thus offset from tether 40 by a few millimeters towards the centerline of the nasal septum (right behind the vomer).

In a further embodiment, device body 32 has a maximum surface area of 6,000, preferably 3,000 square millimeters in order to minimize contact area with airway tissues. For example, the surface area of the device body illustrated in Figure 12a is 1,600 square millimeters and in Figure 12b is 2,400 square millimeters.

In an alternative embodiment, device body 32 can be essentially a straight tube with accordion-like pleats in bent regions 70 and/or 72 that allow for device body 32 to flex and take up to a 90 degree bend in either region. Alternatively, the thin wall in regions 70 and/or 72 can have circumferential or spiral stiffening elements inside or outside the wall that allow for the bending of a thin wall tube without buckling.

Additionally, the material from which at least a portion of tether 40 and/or device body 32 is made can be relatively hard at room temperature and soften at body temperature, which would allow for easy insertion and then a reduction of the forces applied to the airway tissues by device body 32 or tether 40 when inserted into the proper position, thereby increasing tolerability.

In yet a further embodiment, device body 32 and/or tether 40 can include biasing elements that function to fix or bias their radial position in the lumen. For example, device body 32 can be centered using symmetrical elastic biasing elements extending radially from device body 32 in the form of semicircular side struts or curved whiskers that contact the lateral pharyngeal walls and keep device body 32, which is smaller than the diameter of the airway, centered in the airway with a minimum of contact with the airway tissue itself. Such side struts can also be designed to prop open the lateral pharyngeal walls to help prevent airway blockage.

Alternatively, device body 32 can be biased against one side of the lumen (against the lateral pharyngeal wall) using an asymmetrical biasing element or a pre-existing rotational angle between device body 32 and anchor 50 using the torsional stiffness of tether 40 to apply a predetermined biasing force on the device body 32. Such a biasing angle can from 0 (parallel) to 45 degrees. An advantage of biasing device body 32 to the side is that it can be placed lower down the arch of the tonsillar pillar region without being grabbed and swallowed by the base of the tongue in the central higher portion of this "window" behind the uvula. In other words, the device can be positioned to the side of the moving uvula and base of the tongue which leads to increased comfort. Two such device bodies 32 can be biased in the opposite directions against both sides of the lateral pharyngeal walls while still being connected to a common tether 40. In any event, upon airway narrowing during an apnea event, device body 32 is by design pushed to the center of the lumen to provide a minimal patent airway. Alternatively, device body 32 can be aligned on tether 40 relative to anchor 50 so that device body 32 is centered in the lumen behind the uvula so that uvula vibrations against the posterior pharyngeal wall, which are a main cause of snoring, are dampened or eliminated by device body 32. In this manner, device body 32 acts both as a bumper against which the uvula can bump up against, while at the same time the airflow through device body 32 relieves the air pressure differential that causes fluttering and vibration in airway tissues that leads to snoring.

In summary, device body 32 of the present invention is sized to be smaller than the normal airway and resides in the lumen with minimal contact with airway tissue. Contact area with airway tissue is increased mainly during episodes of SDB, but other than in resisting the closure of the last few millimeters of the airway tissues, device body 32 does not resist the natural motions of the airway tissues, including allowing for full velopharnygeal sealing during swallowing, coughing and talking.

By way of contrast, prior art devices that stiffen airway tissue with various implants or stents are in constant contact with the tissue over larger surface areas, limit airway tissue motion, and cause discomfort and eventually ulceration, infection, tissue erosion or explantation of the devices as they are constantly resisting or "fighting" the dynamic nature of these tissues.

Device 30 includes tether 40 which can be designed in a number of ways. Tether 40 can be biased downward against the floor of the nasal cavity using a biasing element pushing gently against tissue in the nasal cavity, for example the inferior nasal conchae or the top of the pharynx. Tether 40 can have a slight downward or upward curvature to control the distance above the nasal cavity floor and the force restoring device body 32 to its desired position if displaced due to movement of the soft palate or other forces. Tether 40 can be comprised of one or more interconnected elements that act as a trussel beam to increase resistance to movement in certain directions. For example, tether 40 can be comprised of two spaced apart axial elements running roughly in parallel and connected at their ends. The bottom element can run along or near the nasal cavity floor and the top element can run to the side or slightly above the inferior turbinate. Such a configuration supplies rigidity and stability with a minimum of structure. Tether 40 or portions thereof can also gently impinge on the turbinates, the top of the nasal cavity, or posterior pharyngeal wall to prevent device body 32 from rising or falling from its desired deployment position.

Tether 40 can be easier to bend in the lateral dimension (to more easily curve around the turbinates or a deviated septum) than in the up and down dimension (to more solidly anchor device body 32 in the pharynx and minimize upward and downward motion of device body 32 in response to swallowing forces). Lateral bending forces of 10-100 grams would displace a 5 cm long section of tether 40 approximately 2-4 cm. The forces required to cause a similar up and down displacement of tether 40 would preferably be 2-10 times as higher than those required for a lateral displacement.

Hinged portion 48 can be a flattened portion of tether 40 such that the hinge only has one rotational degree of freedom that is twisted relative to the plane of tether 40 or anchor 50 to ensure that device body 34 is pointing straight down or at any other desired angle in the airway lumen.

It will be appreciated that if device 30 inadvertently detaches from its anchor site or if device body 32 detaches from tether 40, there is the possibility of device 30 or portions thereof being aspirated or swallowed. To increase safety, all portions of detached device 30 can be made small enough to not fully obstruct an airway if aspirated, and can travel harmlessly through the GI tract and be removed from the body via defecation if swallowed. Furthermore, tether 40 can be made long and rigid enough to not enable it to make the downward curve into the pharynx and therefore render it unswallowable even if anchor 50 should detach from the desired anchoring site. Anchor 50 can also be made large enough (e.g. a ring of around 15 to 25 mm in diameter) such that it cannot enter through the distal end of the nares which is the smallest point of the nasal airway. Anchor 50 can be comprised of elements greater than 5 mm in diameter in at least two planes to further make anchor 50 impassable through the entrance to the nasal cavity. For example, a semicircular-shaped portion of tether 40 emerging from the nose can have a diameter of 5-15mm as tether 40 bends around to the outside of the nostril and then at 90 degrees out of plane the ring portion of anchor 50 that rests against the external nostril tissue can have a diameter of 5-15 mm thereby making anchor 50 5-15 mm in two orthogonal dimensions and therefore un-passable whereas each of the individual flat elements would be passable through the slit-like entrance to the nasal cavity. In a further embodiment, as illustrated in Figure 12 and 13a-c, the nose clip that wraps around the columella serves as anchor 50 and is greater than 5 mm long and 5 mm wide and therefore un-passable through the nasal cavity.

Device 30 can be implanted as a long-term or short-term device. Tether 40 can be attached reversibly or irreversibly to any of the airway tissues between the nostrils and the nasopharyngeal walls, including the nasal septum. Device body 32 can be connected directly to anchor 50 without tether 40. For example, device body 32 can be implanted into posterior pharyngeal wall using a minimally invasive anchor 50 such as a clip, T anchor or suture. Device body 32 can be made of or coated with any material that resists biofilm formation or colonization by bacteria, fungi or other organisms.

The present device can be anchored to any airway tissue using any of the following elements or combinations thereof: elastic or inelastic tethers, t-bar anchors, sutures, button-type anchors, hooks, magnetic clasps, cages, clips and the like. For example, anchoring elements can be delivered through the palatal tissue (centered by the uvula region or on the side by the tonsillar pillar) and anchored thereagainst using T-bar or button type stops preferably fabricated from a polymer such as silicone or a harder material.

Alternatively, tissue penetrating elements can penetrate the target tissue and deliver anchor 50 (which can be attached to device body 32 or tether 40) into the tissue thereby anchoring the device at the correct position. In one embodiment, anchoring is facilitated by a set of one or more needles which are pre-spaced and are introduced through the oral cavity from behind the soft palate or trans-nasally through the nostril. The needles are connected to the anchoring element, which can be a T-bar, barb, button, ball and the like.

Preferable locations to anchor include the nares, the columella (tissue between the nostrils), the fibrous cartilage at front of nasal septum, the mucosal covered cartilage deeper in the septum, the vomer or perpendicular wall of the ethmoid bone in the back of the nasopharynx and the posterior pharyngeal wall.

Device 30 is preferably designed to be introduced and removed by the user on a daily basis. The removed device is either discarded and a new (optionally sterile) one used, or the removed device is cleaned or disinfected for repeated use. In all embodiments described in the present invention, some portion of device 30 such as an external coating can dissolve, detach or degrade after a set amount of time to encourage the replacement of the device, by for example elimination of the colored coating or diffusion or a colorant therein such as a dye, exposing a different colored material underneath the external coating, or by exposing an intolerable or rough surface finish. This approach helps to ensure that no bacteria or fungus colonize the device due to repeated use. Additionally, device body 32 can be modularly attached to tether 40 or anchor 50 to make for a mixed system comprising a reusable element (e.g. anchor 50 and/or tether 40) and a disposable element (e.g. device body 32).

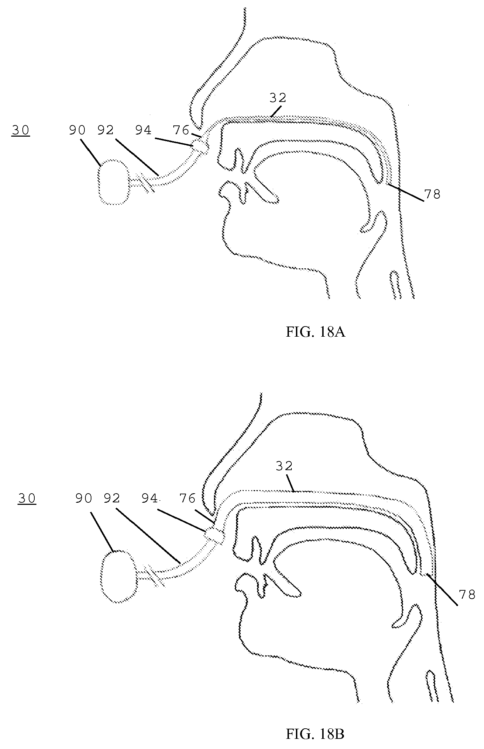

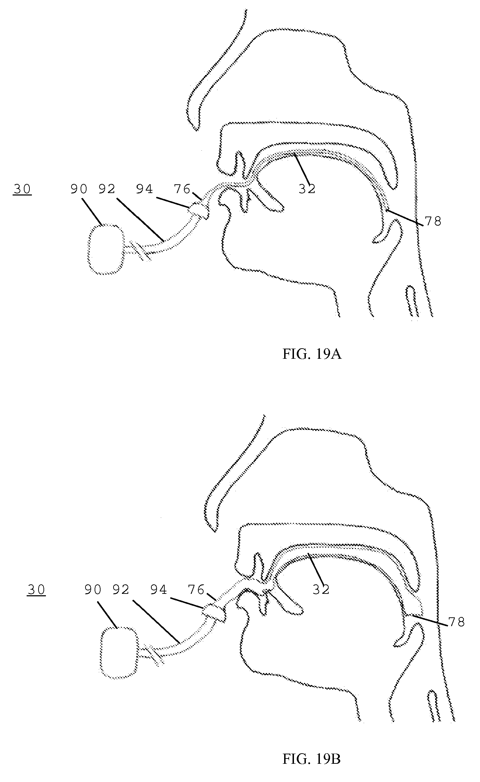

In a further example illustrated in Figures 18a-b and 19a-b, device body 32 is a thin walled lumen-conforming tube (e.g. 1-30 mm in diameter and 0.1 mm wall thickness or less) of insufficient mechanical rigidity to withstand the vacuum forces caused by inspiration without crushing or collapsing, or alternatively a fully collapsed sheath (such as lay flat tubing) of 1-30 mm in diameter made out of a thin and biocompatible polymer (e.g. 0.01 to 0.05 mm thick polyurethane or polyethylene) that is inserted in a collapsed (e.g. flat) state into a nasal cavity (Figure 18a) or the oral cavity (Figure 19a) using a fixed or removable stiffener element 74 (e.g. a 0.1 to 1.0 mm diameter rod of any stiff material such as metals or polymers (e.g. nitinol, nylon, polyurethane or polypropylene), or a water soluble material such as PVA, or a heat sensitive polymer that softens at body temperature which is attached to distal opening 78 of device body 32 or attached along the entire length of device body 32 as an integral assembly. The leading edge of stiffener element 74 or a region of distal opening 78 can be blunt, round and/or soft to minimize trauma of insertion, and optionally removable, detachable or dissolvable to leave just a minimal sheath structure in the airway during use of device 30. Device body 32 itself or portions thereof, including coatings applied to device body 32, can be relatively stiff when dry or at room temperature and soften when in the body, thereby negating the need for stiffener element 74.

Device body 32 can be easily crushed or remain fully flat until inflation and therefore not cause any discomfort as it does not have sufficient thickness to trigger a substantial foreign body response, nor sufficient mechanical integrity to impinge on or resist and motion of airway tissues. Device body 32 can be inflated by external air source 90, such as a compressed gas source or an air pump as found in a standard CPAP machine known in the art using regular, heated and/or humidified air. A positive displacement piston or bellows device can also be used to provide sufficient air in a quiet and gentle manner independent of the resistance to airflow through device body 32. Air source 90 inflates the collapsed device body 32 and passes air beyond the site of the airway obstruction, even as far as the level of the epiglottis to include the full base of the tongue and any blockage in the septum/turbinate region of the nasal cavity. Lower inflation pressures against the tissue relative to standard CPAP should be achievable since the air in device body 32 does not need to break the surface tension of mucosal secretions present in the area of airway blockage, and the tissue that is not in contact with device body 32 is not pressurized at all. Inflation of device body 32 (see Figures 18b and 19b) causes the walls of device body 32 to conform to the contours of airway tissue such as the lumen between the septum and turbinate, or any intervening tissues such as the teeth or lips, yet the air pressure is enough to gently push open these tissues and allow the appropriate air to flow through device body 32 and overcome any blockage that causes SDB. Inflation of device body 32 can occur on a constant basis, ramped up over a period of time after initiation, occur only during inspiration (inhalation), or only when an apnea or snoring event is detected using sensors described elsewhere in this application (e.g. snoring sound, airflow, air pressure, tissue pressure, vibration sensor, mattress motion, motion sensors, peripheral arterial tone sensors, EEG sensors, etc.). Device body 32 can be coated with a hydrophilic, soft or lubricious external coating to make it even more tolerable than the underlying base material. Reduced friction due to a hydrophilic lubricious coating on a flat and deflated device body 32 that passes over the tongue or behind the tongue base helps prevents the tongue from pulling on device body 32 that could cause an uncomfortable gagging or foreign body sensation. Device body 32 can be connected to anchor 50 which can be attached to the nostril region as described previously, or with the assistance of a simple band around the user's head such as used for securing oxygen nasal cannulas. Proximal opening 76 of device body 32 can be connected via connector 94 to the air source directly at anchor 50, or via an external stretch of tubing 92. The advantages of this embodiment over traditional CPAP machines are that there is no need to wear a nose or face mask since no pressure seal is needed on the facial skin surface, there is no unpleasant pressurization of the entire nasal cavity, exhalation can be done effortlessly around device body 32 as the airway normally opens fully during exhalation (most CPAP machines require users to exhale against positive air pressure), and there is a reduction of the size of tubing 92 connecting the user to air source 90. Higher pressure air can also be used relative to CPAP to reduce the diameter of tubing 92 between the air source and device body 32 to 1-10 mm in diameter. The inserted portion of device body 32 can be discarded nightly and a new (and optionally sterile) device body 32 connected to air source 90 at every use.

In an further example device 30 in any embodiment described in the present invention can be used in conjunction with a mandibular advancement device to provide two sources of forces that open obstructed airways. The amount of mandibular advancement necessary when used together with device 30 is likely to be significantly less than a mandibular advancement device alone. Alternatively, for people who sleep with their mouth open, various straps exist to keep the mouth closed during sleep and can be used in conjunction with device 30 to ensure nasal breathing.

It will be appreciated that although the present devices are described in context of treatment of breathing disorders such as apnea, such a device or a modification thereof can also be utilized to treat snoring since it can be used to eliminate or reduce the air pressure differential that causes airway tissues, such as the uvula, to vibrate, or to modify the stiffness or dynamics of the relevant airway tissue and thus dampen the vibration or cushion the impact thereof during sleep related breathing. Furthermore, the present devices can be used as a traditional yet much more tolerated nasopharyngeal airway for medical purposes unrelated to SDB. Therefore, device 30 described herein is useful in reducing snoring as one aspect of sleep disordered breathing, in addition to its utility in treating obstructive sleep apnea and other medical uses of an unobstructed airway through the nasal passageway.

As used herein the term "about" or "approximately" refers to ± 10 %.

It is expected that during the life of this patent many relevant biocompatible materials will be developed and the scope of the materials used in device 30 is intended to include all such new technologies *a priori.*

### Example 1

### The use of Device 30 as per Figure 3-6 in a subject

The device illustrated in Figures 3-6 was constructed and delivered in accordance with the teachings of the present invention and positioned within a nasopharyngeal cavity of a male test subject that suffers from heavy snoring. Device body 32 and tether 40 were in injection molded from polypropylene (G70, Carmel Olefins, Israel) and attached via a hole to anchor 50 which was made of a plastic resin in the form of a 1 mm thick ring 20 mm in diameter. Device body 32 consists of 4 ribs, each 0.6mm in diameter that form two ellipses with an 8 mm minor diameter and 40 mm major diameter and that are offset by 90 degrees from one another. Device body 32 was dip coated three times in RTV silicone (E41, Wacker, Germany) that was further diluted by xylene and allowed to air dry and cure at room temperature. Tether 40 was 1 mm in diameter and 11 cm long and injected molded as one piece in the same mold as device body 32. The proximal end of curved portion 42 of tether 40 was introduced into a hole of anchor 50 and heat welded together. Hinged portion 48 was formed by heat stretching a short section of the 1mm diameter portion of tether 40 until it was 0.4 mm in diameter and 4 mm long.

Device 30 was easily delivered and deployed though the left nostril of the subject without inducing any perceived discomfort; local anesthesia was not required. Anchor 50 remained outside the left nostril and applied gentle pressure on the external skin of the nostril to keep device 30 in place. The subject reported no discomfort while device 30 was in position.

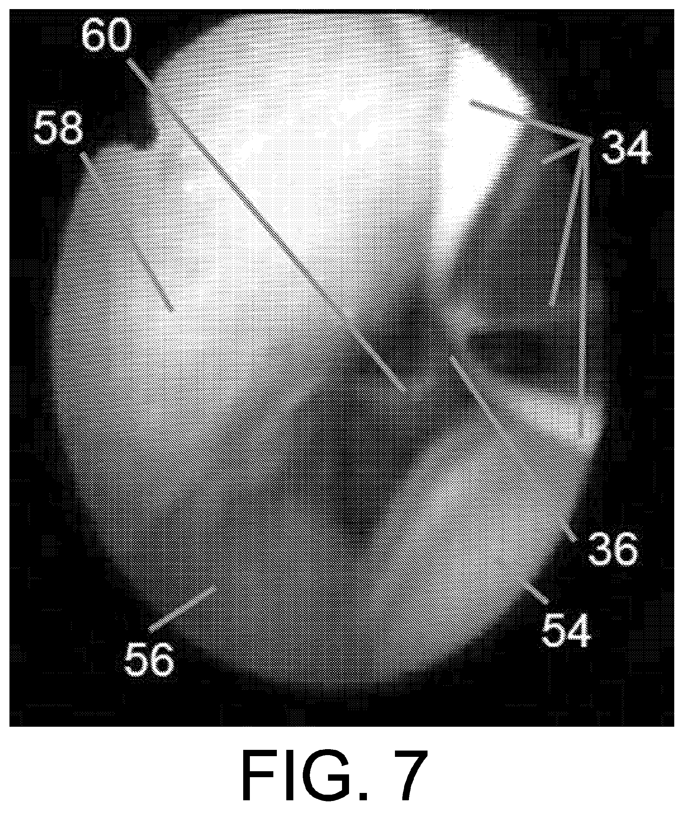

A pediatric nasal endoscope was introduced through the right nostril and used to visualize device body 32 in the nasopharynx from above. The nasal endoscope was positioned in the airway aside device body 32 as illustrated in Figure 7. In Figure 7, four ribs 34 are visible as is the distal end 36 of device body 32. Also visible are soft palate/uvula 54, right lateral pharyngeal wall 56, posterior pharyngeal wall 58 and epiglottis 60. Figure 7 clearly demonstrates that device body 32 does not occupy the entire lumen of the airway. When the subject swallowed or talked, the inner lumen of device body 32 was observed to partially compress between soft palate/uvula 54 and posterior pharyngeal wall 58. Therefore, Figure 7 also demonstrates that device body 32 provides support and prevents collapse in the anterior-posterior axis between soft palate/uvula 54 and posterior pharyngeal wall 58. It will be appreciated that if right lateral pharyngeal wall 56 were to also collapse towards the midline of the lumen, device body 32 would provide support for such collapse due to its position. The subject reported no discomfort during this procedure, even while swallowing or talking.

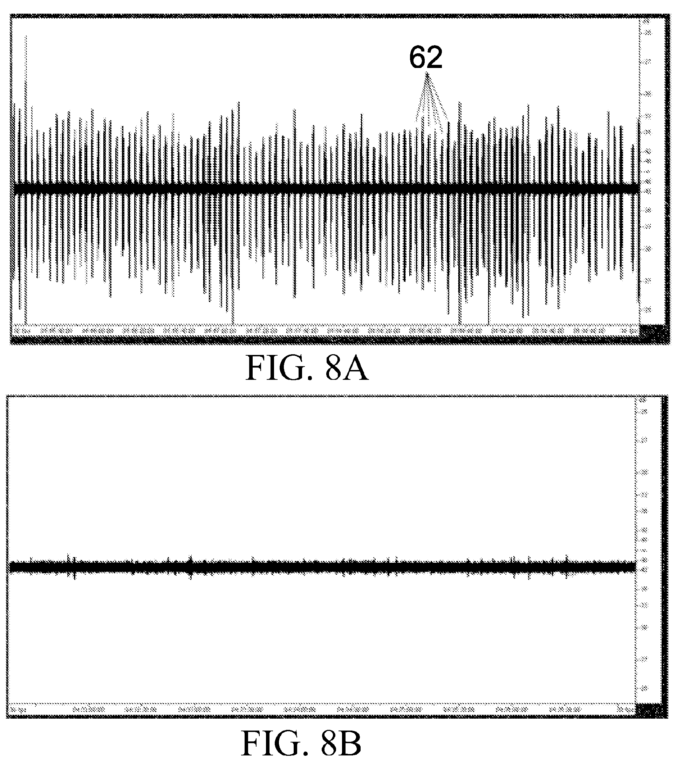

The subject, who suffers from heavy snoring slept one night without device 30 and the sound of snoring was recorded as illustrated in Figure 8a. Individual snores 62 are clearly visible on the trace and audible on the recording. The subject slept the second night with device 30 in place and awoke refreshed in the morning, reported no discomfort from the device and reported that he could have continued wearing the device indefinitely. The sound trace from the second night using the same recording conditions is illustrates in Figure 8b, showing a total cessation of snoring. At the conclusion of the experiment, the subject removed the device by pulling on anchor 50 to pull out device body 32 through the nostril via tether 40. Therefore it was demonstrated that the present device has sufficient force to significantly reduce snoring, maintain a patent airway during sleep and prevent airway collapse, yet device body 32 is crushable enough to maintain velopharyngeal sufficiency during swallowing with high patient tolerability. The example above shows that the present invention successfully addresses the shortcomings of the presently known art by providing an air pathway through collapsed airway tissue during sleep with a device that is safe, non-invasive, reversible, tolerable and effective during both waking and sleeping hours.

### Example 2

### Device configuration having a pre-shaped tubular device body

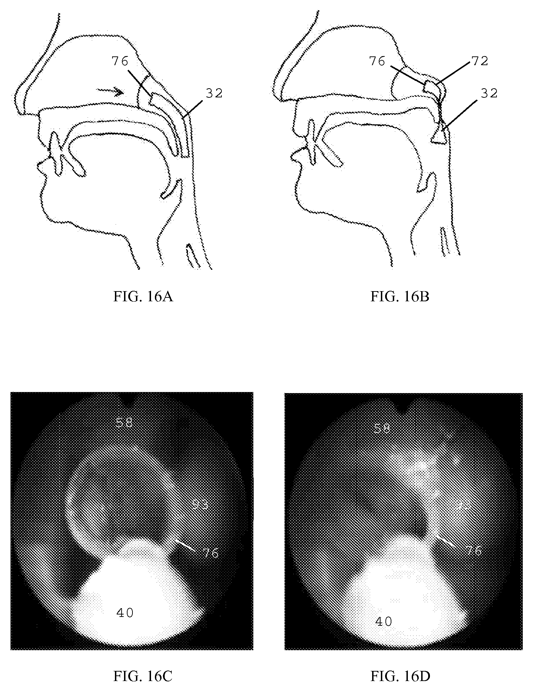

The devices illustrated in Figures 13a-c, 14a-b, 15a-d and 16a-d were constructed and delivered in accordance with the teachings of the present invention and positioned within a nasopharyngeal cavity of a male test subject that suffers from heavy snoring. Tether 40 and anchor 50 were as Figure 13a-c but are not shown for clarity in Figures 15a-b and 16a-b.

In a first embodiment pictured in Figure 15a-d, device body was a straight tube 35 mm long, 6.4 mm in external diameter and wall thickness of 0.25 mm made from molded silicone rubber of Shore A hardness value of 20 that was coated with a hydrophilic outer coat. The resulting device body has a hydrophilic outer surface and a hydrophobic (silicone) inner lumen. Figure 15a illustrates the position of device body 32 in the pharynx during a relaxed state, and Figure 15b illustrates device body 32 in the pharynx during a swallow when soft palate 54 rises to seal off the nasal cavity. Figure 15c is an endoscopic image of device body 32 in the pharynx taken in the direction of the arrow during a relaxed state corresponding to 15a, and Figure 15d is an endoscopic image of device body 32 in the pharynx during a swallow corresponding to Figure 15b. The elevation of soft palate 54 to seal the pharynx is clearly visible in Figures 15d. Proximal opening 76 of device body 32 rested behind nasal septum 91 and against posterior pharyngeal wall 58. Note that in Figure 15b and 15d, proximal opening 76 impinges on and is surrounded by posterior pharyngeal wall 58, soft palate 54, and opening of the Eustachian tube 93, creating significant discomfort as these are very sensitive airway tissues. The test subject did not find this embodiment highly tolerable and it was removed.

In a second embodiment, device body 32 was identical to the above embodiment with the exception of the addition of a curved top section 72 with radius of curvature 73 of 20 mm forming an arc of 50 degrees for a device body 32 length of 40 mm, and angle 75 was 40 degrees, as illustrated in Figure 13a. Tether 40 was made from nylon with a 0.8 mm x 1.6 mm oval cross section over-molded with silicone and attached to the proximal opening 76 of device body 32 via a 1 cm long hinged portion 48 made out of 2 mm OD silicone of shore A 20. Hinged portion 48 enables device body 32 to pivot and assume any angle relative to tether 40 with a force of less than 15 grams. Stiffener element 74 was a 1 mm diameter straight nylon rod that ran freely alongside tether 40 and entered approximately 3.5 cm into device body 32 beyond proximal opening 76. The distal end of stiffener element 74 rested against a little internal step (or sleeve) close to distal opening 78 of device body 32 so that it would not poke beyond distal opening 78 and impinge on airway tissue during device 30 insertion, but was otherwise un-attached to device body 32. Stiffener element 74 bent hinged portion 48 and pivoted device body 32 in the direction of the axis formed by tether 40 for insertion as illustrated in Figure 13c. Anchor 50 was a continuation of tether 40 in a curved shape to fit as a nose clip around columella 51.

Device 30 was easily self-delivered and deployed though the left nostril of the subject without inducing any discomfort; local anesthesia was not required. Anchor 50 was clipped onto columella 51 to keep device 30 in place. The subject reported no discomfort while device 30 was in position.

Figure 16a illustrates device body 32 in the pharynx during a relaxed state, and Figure 16b illustrates device body 32 in the pharynx during a swallow when soft palate 54 rises to seal off the nasal cavity. Figure 16c is an endoscopic image of device body 32 taken in the direction of the arrow in the pharynx during a relaxed state corresponding to 16a, and Figure 16d is an endoscopic image of device body 32 in the pharynx during a swallow corresponding to Figure 16b. The closure of airway tissues during the swallow and the crushing of device body 32 can be seen in Figure 16d through the transparent device body 32 as viewed through proximal opening 76. Proximal opening 76 of device body 32 rested behind nasal septum 91, but due to curved top section 72 did not touch any airway tissues and was suspended in the airway lumen (as opposed to the previous embodiment with a non-curved device body 32 above in which proximal opening 76 rested against posterior pharyngeal wall 58). Note that in Figure 16b and 16d, proximal opening 76 does not touch, impinge on or impact posterior pharyngeal wall 58, soft palate 54, or opening of the Eustachian tube 93. Therefore, this embodiment was comfortable and easily tolerated by the subject.

The subject slept one night without device 30 and the sound of snoring was recorded. The subject slept the second night with device 30 in place and awoke refreshed in the morning, reported no discomfort from the device and reported that he could have continued wearing the device indefinitely. The sound trace from the second night showed a total cessation of snoring similar to Figure 8b. At the conclusion of the experiment, the subject removed the device by pulling on anchor 50 to pull out device body 32 through the nostril via tether 40.

These results clearly demonstrate that the present device has sufficient force to significantly reduce snoring, maintain a patent airway during sleep and prevent airway collapse, yet device body 32 is crushable enough to maintain velopharyngeal sufficiency during swallowing with high patient tolerability, due in part to curved section 72 that allows for proximal opening 76 to not impinge on sensitive airway tissues.

It is appreciated that certain features of the invention, which are, for clarity, described in the context of separate embodiments, may also be provided in combination in a single embodiment. For example, any device body, tethering, anchoring or delivery embodiment, configuration or scheme can be combined with each other to create a device that achieves all of the functions described herein. Conversely, various features of the invention, which are, for brevity, described in the context of a single embodiment, may also be provided separately or in any suitable subcombination.

## Claims

1. A device for treating sleep disordered breathing comprising a tether (40) attached to a device body **characterized in that** the device body is configured as a pre-shaped tube (32) having a proximal curved portion (72) with a proximal opening (76) and distal straight portion (80) with a distal opening (78), wherein an angle (75) between said curved portion (72) and said straight portion (80) is between 30 to 90 degrees and further wherein said proximal curved portion (72) has a radius of curvature of 40 mm or less so as to match a curvature of a pharyngeal lumen along the resting soft palate.

2. The device of claim 1, wherein said tube (32) is configured such that it does not radially collapse closed during involuntary collapse of said pharyngeal lumen.

3. The device of claim 1, wherein said at least a portion of said tether (40) suspends said proximal opening (76) of said tube (32) above a descending soft palate.

4. The device of claim 3, wherein said proximal opening (76) of said tube (32) rests just behind a distal edge of a nasal septum or vomer.

5. The device of claim 1, wherein at least a portion of said tether (40) is stiff.

6. The device of claim 5, wherein at least a portion of said tether (40) rests on a floor of said nasal passageway when the device is in use.

7. The device of claim 5, wherein a proximal end of said tether (40) is attached to tissue between nostrils or nares.

## Patentansprüche

1. Vorrichtung zur Behandlung von Atemstörungen im Schlaf, umfassend einen Haltegurt (40), der an einen Vorrichtungskörper befestigt ist, **dadurch gekennzeichnet, dass** der Vorrichtungskörper als ein vorgeformtes Rohr (32) konfiguriert ist, umfassend einen proximalen gekrümmten Abschnitt (72) mit einer proximalen Öffnung (76) und einen distalen geraden Abschnitt (80) mit einer distalen Öffnung (78), wobei ein Winkel (75) zwischen dem gekrümmten Abschnitt (72) und dem geraden Abschnitt (80) zwischen 30 und 90 Grad liegt, und weiter wobei der proximale gekrümmte Abschnitt (72) einen Krümmungsradius von 40 mm oder weniger aufweist, um zu einer Krümmung eines pharyngealen Lumens entlang des ruhenden weichen Gaumens zu passen.

2. Vorrichtung nach Anspruch 1, wobei das Rohr (32) derart konfiguriert ist, dass es während eines unwillkürlichen Zusammenbruchs des pharyngealen Lumens nicht radial in den geschlossenen Zustand zusammenbricht.

3. Vorrichtung nach Anspruch 1, wobei mindestens ein Abschnitt des Haltegurts (40) die proximale Öffnung (76) des Rohrs (32) über einem absteigenden weichen Gaumen aussetzt.

4. Vorrichtung nach Anspruch 3, wobei die proximale Öffnung (76) des Rohrs (32) genau hinter einem distalen Rand eines nasalen Septums oder Pflugscharbeins aufliegt.

5. Vorrichtung nach Anspruch 1, wobei mindestens ein Abschnitt des Haltegurts (40) steif ist.

6. Vorrichtung nach Anspruch 5, wobei mindestens ein Abschnitt des Haltegurts (40) auf einem Boden des nasalen Durchgangs aufliegt, wenn die Vorrichtung verwendet wird.

7. Vorrichtung nach Anspruch 5, wobei ein proximales Ende des Haltegurts (40) an Gewebe zwischen Nasenöffnungen oder und Nasenlöcher befestigt ist.

## Revendications

1. Dispositif pour traiter la respiration affectée par des troubles du sommeil, comprenant une attache (40) fixée à un corps de dispositif, **caractérisé en ce que** le corps dispositif est configuré comme un tube préformé (32) ayant une partie proximale incurvée (72) avec une ouverture proximale (76) et une partie distale droite (80) avec une ouverture distale (78), dans lequel un angle (75) entre ladite partie incurvée (72) et ladite partie droite (80) est compris entre 30 et 90 degrés et en outre dans lequel ladite partie proximale incurvée (72) a un rayon de courbure de 40 mm ou moins afin de correspondre à une courbure d'une lumière pharyngée le long du voile du palais au repos.

2. Dispositif selon la revendication 1, dans lequel ledit tube (32) est configuré de sorte qu'il ne se ferme pas par affaissement radial pendant le collapsus involontaire de ladite lumière pharyngée.

3. Dispositif selon la revendication 1, dans lequel ladite au moins une partie de ladite attache (40) suspend ladite ouverture proximale (76) dudit tube (32) au-dessus d'un voile du palais descendant.

4. Dispositif selon la revendication 3, dans lequel ladite ouverture proximale (76) dudit tube (32) s'appuie juste derrière un bord distal d'une cloison nasale ou du vomer.

5. Dispositif selon la revendication 1, dans lequel au moins une partie de ladite attache (40) est rigide.

6. Dispositif selon la revendication 5, dans lequel au moins une partie de ladite attache (40) s'appuie sur un plancher de ladite voie de passage nasale lorsque le dispositif est utilisé.

7. Dispositif selon la revendication 5, dans lequel une extrémité proximale de ladite attache (40) est fixée sur le tissu entre les narines.
